# EUROPEAN PATENT APPLICATION

(11) **EP 4 701 282 A1**
(43) Date of publication of application: **25.02.2026**
(21) Application number: 24864316.5
(22) Date of filing: 02.08.2024
(51) Int. Cl.: H04W 52/02

(54) **SERVICE PROCESSING METHOD AND RELATED APPARATUS**

(30) Priority: 15.09.2023 CN 202311193006
(71) Applicant: Huawei Technologies Co., Ltd., Shenzhen, Guangdong 518129 (CN)
(72) Inventor: CAI, Kunlun, Shenzhen, Guangdong 518129 (CN)
(74) Representative: Thun, Clemens
(86) International application number: PCT/CN2024/109612
(87) International publication number: WO 2025/055593

(57) **Abstract**

This application provides a service processing method and a related apparatus, and may be applied to the field of terminal technologies. In this application, a terminal device may determine, at a first moment, whether a first service and a second service are in an enabled state, periodically execute the first service when the first service is in the enabled state, and periodically execute the second service when the second service is in the enabled state, where the first service is a service for periodically enabling a first sensor to collect data, the second service is a service for periodically enabling a second sensor to collect data, and both the first sensor and the second sensor may include a target sensor. According to the method in this application, execution time of the first service and execution time of the second service may overlap at a specific moment. In this way, the target sensor needs to be enabled only once at the specific moment, so that a quantity of times of enabling the target sensor can be reduced, thereby helping reduce power consumption of the terminal device.

## Description

This application claims priority to Chinese Patent Application No. 202311193006.0, filed with the China National Intellectual Property Administration on September 15, 2023 and entitled "SERVICE PROCESSING METHOD AND RELATED APPARATUS", which is incorporated herein by reference in its entirety.

### TECHNICAL FIELD

This application relates to the field of terminal technologies, and in particular, to a service processing method and a related apparatus.

### BACKGROUND

With development of terminal devices, users have more function requirements for the terminal devices. For example, the terminal devices may be configured to execute a plurality of periodic services used for health detection, for example, periodic blood oxygen and periodic heart rate.

Currently, a method for executing a periodic service by a terminal device may include: After receiving an instruction of a user for enabling the periodic service, the terminal device may periodically control, based on an execution periodicity of the periodic service, a sensor related to the periodic service to collect data.

However, in a use process, it is found that when the terminal device executes a plurality of periodic services, power consumption of the terminal device is high.

### SUMMARY

This application provides a service processing method and a related apparatus, to resolve a problem of high power consumption of a terminal device in a conventional technology.

According to a first aspect, this application provides a service processing method. The method is applied to a terminal device. The method includes: determining, at a first moment, whether a first service is in an enabled state, where the first service is a service for periodically enabling a first sensor to collect data, an execution periodicity of the first service is a first periodicity, and the first sensor includes a target sensor; if the first service is in the enabled state at the first moment, periodically executing the first service by using the first moment as a start moment; determining, at the first moment, whether a second service is in an enabled state, where the second service is a service for periodically enabling a second sensor to collect data, an execution periodicity of the second service is a second periodicity, and the second sensor includes the target sensor; and if the second service is in the enabled state at the first moment, periodically executing the second service by using the first moment as a start moment.

In the method, the first moment may be preset in advance. For example, the first moment may be 0: 10.

Optionally, the first moment may alternatively be a power-on moment of the terminal device. For example, assuming that the terminal device is powered on at 0:10, the first moment may be 0:10.

Optionally, a duration between the first moment and the power-on moment of the terminal device may alternatively be a preset duration. The preset duration may be preset in advance. For example, assuming that the terminal device is powered on at 0:10, and the preset duration is five minutes, the first moment may be 0:15.

Optionally, the execution periodicity of the first service may also be preset in advance. Optionally, the execution periodicity of the second service may also be preset in advance.

In the method, execution time of the first service and execution time of the second service may overlap at a specific moment. A duration between the specific moment and the first moment may be a common multiple of the first periodicity and the second periodicity.

In this way, the target sensor needs to be enabled only once at the specific moment, so that a quantity of times of enabling the target sensor can be reduced, thereby helping reduce power consumption of the terminal device.

In some possible implementations, the second periodicity is greater than the first periodicity, and the method further includes: if the first service is not in the enabled state at the first moment, determining, at a second moment, whether the first service is in the enabled state, where a duration between the second moment and the first moment is equal to a first duration, and the first duration is equal to a greatest common divisor of a duration of the first periodicity and a duration of the second periodicity.

In an example, assuming that the first periodicity is 10 minutes, and the second periodicity is 30 minutes, the first duration may be 10 minutes. Assuming that the first moment is 0:10, the second moment may be 0:20.

In this example, the greatest common divisor of the duration of the first periodicity and the duration of the second periodicity is equal to the duration of the first periodicity, and the duration between the second moment and the first moment may be equal to the duration of the first periodicity. In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the second moment and the first moment may be equal to the duration of the first periodicity. In this way, a time interval between two consecutive times of determining, by the terminal device, whether the first service is in the enabled state may be equal to the duration of the first periodicity. This helps ensure a value output rate of the first service.

In another example, assuming that the first periodicity is 10 minutes, and the second periodicity is 15 minutes, the first duration may be five minutes. Assuming that the first moment is 0:10, the second moment may be 0:15.

In this example, the duration between the second moment and the first moment may be equal to the greatest common divisor of the duration of the first periodicity and the duration of the second periodicity. In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the second moment and the first moment may be less than the duration of the first periodicity. In this way, a time interval between two consecutive times of determining, by the terminal device, whether the first service is in the enabled state may be less than the duration of the first periodicity. This helps ensure a value output rate of the first service.

In some possible implementations, the method further includes: if the second service is not in the enabled state at the first moment, determining, at a third moment, whether the second service is in the enabled state; and if the second service is in the enabled state at the third moment, periodically executing the second service by using the third moment as a start moment.

Optionally, a duration between the third moment and the first moment may alternatively be equal to the first duration.

In some examples, assuming that the first periodicity is 10 minutes, and the second periodicity is 30 minutes, the first duration may be 10 minutes. Assuming that the first moment is 0:10, the second moment may be 0:20, and the third moment may also be 0:20.

In this example, the third moment and the second moment may be a same moment. In this way, when neither the first service nor the second service is in the enabled state at the first moment, whether the first service and the second service are in the enabled state may be determined at the second moment (or the third moment). When it is determined that the first service and the second service are in the enabled state at the second moment (or the third moment), each service may be periodically executed based on an execution periodicity of each service.

In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the third moment and the first moment may be equal to the duration of the first periodicity, and the duration of the first periodicity is less than the duration of the second periodicity. In this way, a time interval between two consecutive times of determining, by the terminal device, whether the second service is in the enabled state may be less than the duration of the second periodicity. This helps ensure a value output rate of the second service.

In some other examples, assuming that the first periodicity is 10 minutes, and the second periodicity is 15 minutes, the first duration may be five minutes. Assuming that the first moment is 0:10, the second moment may be 0:15, and the third moment may be 0:15.

In this example, the third moment and the second moment may be a same moment. In this way, when neither the first service nor the second service is in the enabled state at the first moment, whether the first service and the second service are in the enabled state may be determined at the second moment (or the third moment). When it is determined that the first service and the second service are in the enabled state at the second moment (or the third moment), each service may be periodically executed based on an execution periodicity of each service.

In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the third moment and the first moment may be less than the duration of the first periodicity, and the duration of the first periodicity is less than the duration of the second periodicity. In this way, a time interval between two consecutive times of determining, by the terminal device, whether the second service is in the enabled state may be less than the duration of the second periodicity. This helps ensure a value output rate of the second service.

In some possible implementations, the method further includes: if the second service is not in the enabled state at the third moment and it is determined that the second service is not in the enabled state at each of M moments, determining, at a fourth moment, whether the second service is in the enabled state, where an interval between the fourth moment and the third moment is a duration of one second periodicity, M is a positive integer, a duration of an interval between an m^{th} moment in the M moments and the first moment is equal to m first durations, m is a positive integer and ranges from 1 to M, an M^{th} moment in the M moments is before the fourth moment, an (M+1)^{th} moment is at the fourth moment, and a duration of an interval between the (M+1)^{th} moment and the first moment is equal to (M+1) first durations.

In an example, assuming that the execution periodicity of the first service is 10 minutes, and the execution periodicity of the second service is 30 minutes, the first duration may be 10 minutes. Assuming that the first moment is 0:10, if the terminal device determines, at 0:10, that the second service is not in the enabled state, the terminal device re-determines, at 0:20, whether the second service is in the enabled state; if the terminal device determines, at 0:20, that the second service is not in the enabled state, the terminal device re-determines, at 0:30, whether the second service is in the enabled state; if the terminal device determines, at 0:30, that the second service is not in the enabled state, the terminal device re-determines, at 0:40, whether the second service is in the enabled state; and if the terminal device determines, at 0:40, that the second service is not in the enabled state, the terminal device re-determines, at 0:50, whether the second service is in the enabled state.

In this example, 0:20 may be the third moment, the M moments may include three moments: 0:20, 0:30, and 0:40, the fourth moment may include 0:50, and the fourth moment is the (M+1)^{th} moment.

In another example, assuming that the execution periodicity of the first service is 10 minutes, and the execution periodicity of the second service is 15 minutes, the first duration may be five minutes. Assuming that the first moment is 0:10, if the terminal device determines, at 0:10, that the second service is not in the enabled state, the terminal device re-determines, at 0:15, whether the second service is in the enabled state; if the terminal device determines, at 0:15, that the second service is not in the enabled state, the terminal device re-determines, at 0:20, whether the second service is in the enabled state; if the terminal device determines, at 0:20, that the second service is not in the enabled state, the terminal device re-determines, at 0:25, whether the second service is in the enabled state; and if the terminal device determines, at 0:25, that the second service is not in the enabled state, the terminal device re-determines, at 0:30, whether the second service is in the enabled state.

In this example, 0:15 may be the third moment, the M moments may include three moments: 0:15, 0:20, and 0:25, the fourth moment may include 0:30, and the fourth moment is the (M+1)^{th} moment.

In the method, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the first duration may be less than or equal to the duration of the first periodicity, and the duration of the first periodicity is less than the duration of the second periodicity. In this way, a time interval between two consecutive times of determining, by the terminal device, whether the second service is in the enabled state may be less than the duration of the second periodicity. This helps ensure a value output rate of the second service.

In some possible implementations, the method further includes: if both the first service and the second service are in the enabled state at a target moment, determining a priority of the first service and a priority of the second service based on at least one of a first condition, a second condition, and a third condition, where the first condition is that a service that requires a larger quantity of sensors has a higher priority, the second condition is that a service with more preset data reporting frequencies of a sensor has a higher priority, the third condition is that a service with a shorter preset service running duration has a higher priority, a determining priority of the first condition is higher than a determining priority of the second condition, and the determining priority of the second condition is higher than a determining priority of the third condition; and determining an execution rank of the first service and an execution rank of the second service in descending sequence of the priority of the first service and the priority of the second service.

Optionally, a sensor required by each service may be preset in advance.

Optionally, a preset reporting frequency, of a sensor, preset for each service may alternatively be preset in advance.

Optionally, a preset service running duration of each service may alternatively be preset in advance.

In a first example, assuming that a quantity of first sensors is greater than a quantity of second sensors, and when a preset reporting frequency of a sensor and a preset service running duration of the first service is the same as a preset reporting frequency of a sensor and a preset service running duration of the second service, the priority of the first service is higher than the priority of the second service, and the execution rank of the first service is higher than the execution rank of the second service.

In a second example, assuming that when a preset reporting frequency of a sensor of the first service is greater than a preset reporting frequency of a sensor of the second service, a quantity of first sensors is the same as a quantity of second sensors, and a preset service running duration of the first service is also the same as a preset service running duration of the second service, the priority of the first service is higher than the priority of the second service, and the execution rank of the first service is higher than the execution rank of the second service.

In a third example, assuming that when a preset service running duration of the first service is less than a preset service running duration of the second service, a quantity of first sensors is the same as a quantity of second sensors, and a preset reporting frequency of a sensor of the first service is also the same as a preset reporting frequency of a sensor of the second service, the priority of the first service is higher than the priority of the second service, and the execution rank of the first service is higher than the execution rank of the second service.

In the method, the terminal device may first execute the first service, and then execute the second service. When executing the first service, the terminal device may enable the first sensor; and when executing the second service, the terminal device may not need to repeatedly enable the shared target sensor. In this way, a quantity of times of enabling the target sensor can be reduced, thereby helping reduce power consumption of the terminal device.

In some possible implementations, determining the execution rank of the first service and the execution rank of the second service in descending sequence of the priority of the first service and the priority of the second service includes: if the priority of the first service is higher than the priority of the second service, determining to enable the first sensor at the target moment, and enable a sensor in the second sensor other than the target sensor after the target moment; or if the priority of the first service is lower than the priority of the second service, determining to enable the second sensor at the target moment, and enable a sensor in the first sensor other than the target sensor after the target moment.

In the method, the terminal device may first enable a sensor required by a service with a high priority, and then enable a sensor in sensors required by a service with a low priority other than the target sensor. In this way, a quantity of times of enabling the target sensor can be reduced, thereby helping reduce power consumption of the terminal device.

In some possible implementations, the method further includes: determining a target data reporting frequency based on a preset data reporting frequency of the first service and a preset data reporting frequency of the second service, where the target data reporting frequency is equal to a greatest common divisor of the preset data reporting frequency of the first service and the preset data reporting frequency of the second service; and determining the target data reporting frequency as a data reporting frequency of the target sensor after the target moment.

In an example, assuming that a preset reporting frequency of a sensor of the first service is reporting once per 50 milliseconds, and a preset reporting frequency of a sensor of the second service is reporting once per 500 milliseconds, the greatest common divisor of the preset data reporting frequency of the first service and the preset data reporting frequency of the second service is reporting once per 50 milliseconds, and the target data reporting frequency may be reporting once per 50 milliseconds.

In another example, assuming that a preset reporting frequency of a sensor of the first service is reporting once per 50 milliseconds, and a preset reporting frequency of a sensor of the second service is reporting once per 60 milliseconds, the greatest common divisor of the preset data reporting frequency of the first service and the preset data reporting frequency of the second service is reporting once per 10 milliseconds, and the target data reporting frequency may be reporting once per 10 milliseconds.

In the method, the data reporting frequency of the target sensor after the target moment may be the target data reporting frequency. In this way, it can be ensured that the data reporting frequency of the target sensor after the target moment can meet both a data reporting frequency requirement of the first service and a data reporting frequency requirement of the second service.

In some possible implementations, the method further includes: determining a target service from the first service and the second service, where the target service is a service with a shorter preset service running duration in the first service and the second service; if a preset service running duration of the target service is shorter than a preset service running duration of another service that is not the target service in the first service and the second service, determining an end moment of the target service based on the preset service running duration of the target service, and an interval between the end moment and the target moment is equal to a preset service duration of the target service; and determining a preset data reporting frequency of the another service as a data reporting frequency of the target sensor after the end moment.

For example, assuming that the preset service running duration of the first service is 1 second, the preset service running duration of the second service is 3 seconds, and the target moment is 0:30, the terminal device determines that the data reporting frequency of the target sensor after 0:30 is the target data reporting frequency, and determines that the data reporting frequency of the target sensor after 0:31 is the preset data reporting frequency of the second service.

In this example, it can be avoided that the target sensor collects and reports some redundant data when the target sensor still performs reporting at a high data reporting frequency after running of the first service is completed, thereby helping further reduce power consumption of the terminal device.

According to a second aspect, this application provides a service processing method. The method is applied to a terminal device. The method includes: A processor of the terminal device determines that a to-be-processed service is in an enabled state at a target moment, where the to-be-processed service is a service for periodically enabling a third sensor to collect data, and the to-be-processed service includes a first service and/or a second service; and when determining to execute the to-be-processed service, the processor sends indication information to the third sensor, where the indication information indicates the third sensor to collect and report the data.

In the method, the processor of the terminal device may be a single-core processor. A method for determining, by the terminal device, whether to enable the to-be-processed service and a method for determining, by the terminal device, to execute the to-be-processed service are both performed by the processor. When the to-be-processed service includes the first service and the second service, execution time of the first service and execution time of the second service may overlap in specific time. In this way, not only power consumption of the terminal device can be reduced, but also response time of executing the to-be-processed service by the terminal device can be reduced, thereby improving a response speed of the to-be-processed service.

In some possible implementations, when the to-be-processed service includes the first service and the second service, the third sensor includes a first sensor and a second sensor, the first service is a service for periodically enabling the first sensor to collect data, the second service is a service for periodically enabling the second sensor to collect data, and both the first sensor and the second sensor include a target sensor; and
that the processor sends the indication information to the third sensor includes: the processor sends the indication information to the first sensor and the second sensor based on a priority of the first service and a priority of the second service.

In some possible implementations, that the processor sends the indication information to the first sensor and the second sensor based on the priority of the first service and the priority of the second service includes:

If the priority of the first service is higher than the priority of the second service, the processor sends first indication information to the first sensor, where the first indication information indicates the first sensor to collect first data and upload the first data at the target moment; and the processor sends second indication information to a sensor in the second sensor other than the target sensor, where the second indication information indicates the another sensor to collect second data and upload the second data after the target moment; or
if the priority of the first service is lower than the priority of the second service, the processor sends third indication information to the second sensor, where the third indication information indicates the second sensor to collect second data and upload the second data at the target moment; and the processor sends fourth indication information to a sensor in the first sensor other than the target sensor, where the fourth indication information indicates the another sensor to collect first data and upload the first data after the target moment.

In some possible implementations, the method further includes: The processor sends fifth indication information to the target sensor, where the fifth indication information indicates the target sensor to report data based on a target data reporting frequency after the target moment, and the target data reporting frequency is equal to a greatest common divisor of a preset data reporting frequency of the first service and a preset data reporting frequency of the second service.

In some possible implementations, the method further includes: The processor sends sixth indication information to the target sensor, where the sixth indication information indicates the target sensor to report data based on a preset data reporting frequency of another service after an end moment of a target service, the target service is a service with a shorter preset service running duration in the first service and the second service, and the another service is a service in the first service and the second service other than the target service.

According to a third aspect, this application provides a service processing method. The method is applied to a terminal device. The method includes: A first processor of the terminal device receives service state information, where the service state information indicates that a to-be-processed service is in an enabled state at a target moment, the to-be-processed service is a service for periodically enabling a third sensor to collect data, and the to-be-processed service includes a first service and/or a second service; when determining to execute the to-be-processed service, the first processor sends first information to a second processor, where the first information indicates to execute the to-be-processed service; the first processor receives indication information from the second processor, where the indication information indicates the third sensor to collect and report the data; and the first processor sends the indication information to the third sensor.

In the method, the processor of the terminal device may be a dual-core processor. A method for determining, by the terminal device, whether to enable the to-be-processed service and a method for determining, by the terminal device, to execute the to-be-processed service are both performed by the first processor. Because power consumption of the first processor is lower than power consumption of the second processor, power consumption of the terminal device can be further reduced.

In some possible implementations, when the to-be-processed service includes the first service and the second service, the third sensor includes a first sensor and a second sensor, the first service is a service for periodically enabling the first sensor to collect data, the second service is a service for periodically enabling the second sensor to collect data, and both the first sensor and the second sensor include a target sensor; and
that the first processor sends the indication information to the third sensor includes: the first processor sends the indication information to the first sensor and the second sensor based on a priority of the first service and a priority of the second service.

In some possible implementations, that the first processor sends the indication information to the first sensor and the second sensor based on the priority of the first service and the priority of the second service includes:

If the priority of the first service is higher than the priority of the second service, the first processor sends first indication information to the first sensor, where the first indication information indicates the first sensor to collect first data and upload the first data at the target moment; and the first processor sends second indication information to a sensor in the second sensor other than the target sensor, where the second indication information indicates the another sensor to collect second data and upload the second data after the target moment; or
if the priority of the first service is lower than the priority of the second service, the first processor sends third indication information to the second sensor, where the third indication information indicates the second sensor to collect second data and upload the second data at the target moment; and the first processor sends fourth indication information to a sensor in the first sensor other than the target sensor, where the fourth indication information indicates the another sensor to collect first data and upload the first data after the target moment.

In some possible implementations, the method further includes: The first processor sends fifth indication information to the target sensor, where the fifth indication information indicates the target sensor to report data based on a target data reporting frequency after the target moment, and the target data reporting frequency is equal to a greatest common divisor of a preset data reporting frequency of the first service and a preset data reporting frequency of the second service.

In some possible implementations, the method further includes: The first processor sends sixth indication information to the target sensor, where the sixth indication information indicates the target sensor to report data based on a preset data reporting frequency of another service after an end moment of a target service, the target service is a service with a shorter preset service running duration in the first service and the second service, and the another service is a service in the first service and the second service other than the target service.

According to a fourth aspect, this application provides an apparatus. The apparatus includes a module or a unit configured to implement the method according to any one of the first aspect and the possible implementations of the first aspect. It should be understood that each module or unit may implement a corresponding function by executing a computer program.

According to a fifth aspect, this application provides an apparatus. The apparatus includes a module or a unit configured to implement the method according to any one of the second aspect and the possible implementations of the second aspect. It should be understood that each module or unit may implement a corresponding function by executing a computer program.

According to a sixth aspect, this application provides an apparatus. The apparatus includes a module or a unit configured to implement the method according to any one of the third aspect and the possible implementations of the third aspect. It should be understood that each module or unit may implement a corresponding function by executing a computer program.

According to a seventh aspect, this application provides an apparatus. The apparatus includes a processor, and the processor is configured to perform the method according to any one of the first aspect to the third aspect and the possible implementations of the first aspect to the third aspect.

The apparatus may further include a memory, configured to store instructions and data. The memory is coupled to the processor; and when executing the instructions stored in the memory, the processor can implement the method described in the foregoing aspects. The apparatus may further include a communication interface. The communication interface is used by the apparatus to communicate with another device. For example, the communication interface may be a transceiver, a circuit, a bus, a module, or another type of communication interface.

According to an eighth aspect, this application provides a computer-readable storage medium. The computer-readable storage medium stores program code to be executed by a service processing apparatus, and the program code includes instructions used to implement the method according to the first aspect to the third aspect.

According to a ninth aspect, this application provides a computer program product including instructions. When the computer program product is run on a service processing apparatus, the service processing apparatus is enabled to implement the method according to the first aspect to the third aspect.

It may be understood that for effect that can be obtained in the second aspect to the ninth aspect, refer to the descriptions in the first aspect. Details are not described herein.

### BRIEF DESCRIPTION OF DRAWINGS

FIG. 1 is a diagram of an intelligent detection scenario to which embodiments of this application are applicable;
FIG. 2 is a diagram of a structure of a terminal device according to an embodiment of this application;
FIG. 3 is a diagram of a user interface according to an embodiment of this application;
FIG. 4 is a diagram of execution time of different services;
FIG. 5 is a diagram of a system architecture of a terminal device according to an embodiment of this application;
FIG. 6 is a diagram of a system architecture of another terminal device according to an embodiment of this application;
FIG. 7 is a schematic flowchart of a service processing method according to this application;
FIG. 8 is a diagram of a plurality of services according to an embodiment of this application;
FIG. 9 is a diagram of scheduling of a plurality of services according to an embodiment of this application;
FIG. 10 is a schematic flowchart of a service processing method for a terminal device according to another embodiment of this application;
FIG. 11 is a schematic flowchart of a service processing method for a terminal device according to still another embodiment of this application;
FIG. 12 is a diagram of a structure of a service processing apparatus according to an embodiment of this application;
FIG. 13 is a diagram of a structure of a service processing apparatus according to another embodiment of this application;
FIG. 14 is a diagram of a structure of a service processing apparatus according to still another embodiment of this application; and
FIG. 15 is a diagram of a structure of a service processing apparatus according to still another embodiment of this application.

### DESCRIPTION OF EMBODIMENTS

The following describes technical solutions in embodiments of this application with reference to accompanying drawings in embodiments of this application.

To clearly describe the technical solutions in embodiments of this application, terms such as "first" and "second" are used in embodiments of this application to distinguish between same items or similar items that have basically the same functions and purposes. For example, first information and second information are merely used to distinguish between different information, and do not limit a sequence of the first information and the second information. A person skilled in the art may understand that the terms such as "first" and "second" do not limit a quantity or an execution sequence, and the terms such as "first" and "second" do not indicate a definite difference.

In embodiments of this application, "at least one" means one or more, and "a plurality of" means two or more. The term "and/or" describes an association relationship between associated objects, and indicates that three relationships may exist. For example, A and/or B may indicate the following cases: Only A exists, both A and B exist, and only B exists, where A and B may be singular or plural. The character "/" usually indicates an "or" relationship between associated objects. "At least one of the following items (pieces)" or a similar expression thereof means any combination of these items, including any combination of singular items (pieces) or plural items (pieces). For example, at least one item (piece) of a, b, or c may indicate a, b, c, a and b, a and c, b and c, or a, b, and c, where a, b, and c each may be singular or plural.

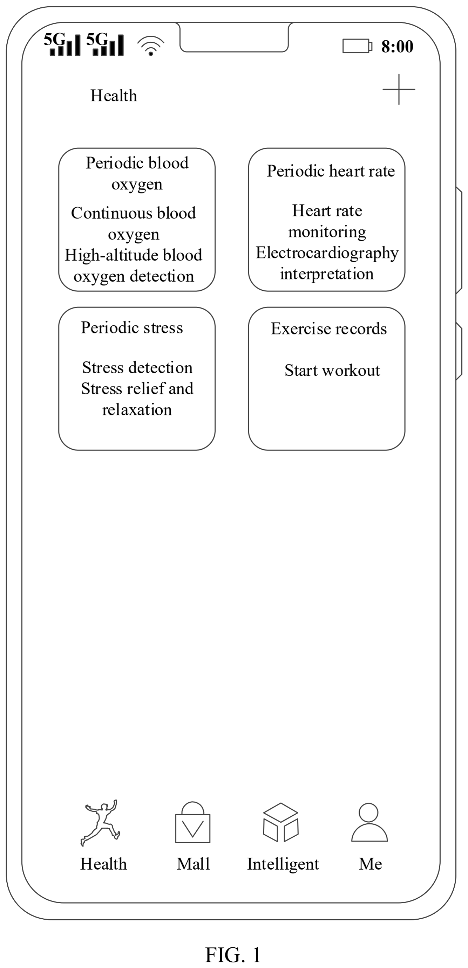

This application may be applied to an intelligent detection scenario shown in FIG. 1. As shown in FIG. 1, a terminal device may be configured to execute a plurality of periodic services, for example, periodic blood oxygen, periodic heart rate, periodic stress, and exercise records.

Each periodic service has a corresponding execution periodicity. For any periodic service, the periodic service is periodically executed based on an execution periodicity of the periodic service.

For example, assuming that an execution periodicity of the periodic blood oxygen is 10 minutes, after a user enables a blood oxygen measurement service on the terminal device, the terminal device may periodically measure blood oxygen, and a time interval between any two adjacent measurements is 10 minutes.

In this application, the terminal device may be a wearable terminal device (also referred to as a wearable device), for example, a watch, a band, a headset, or a helmet (for example, a virtual reality helmet), or may be a non-wearable device, for example, a portable terminal device, for example, a mobile phone, a tablet computer, or a notebook computer, having a detection function such as an accelerometer (accelerometer, ACC), a photoplethysmography (photoplethysmography, PPG), or an electrocardiography (electrocardiography, ECG). An example embodiment of the portable terminal device includes but is not limited to a portable terminal device using another operating system. It should be understood that the terminal device may not be a portable terminal device, but a desktop computer that can detect ECG and/or PPG, or the like. This is not limited in embodiments of this application.

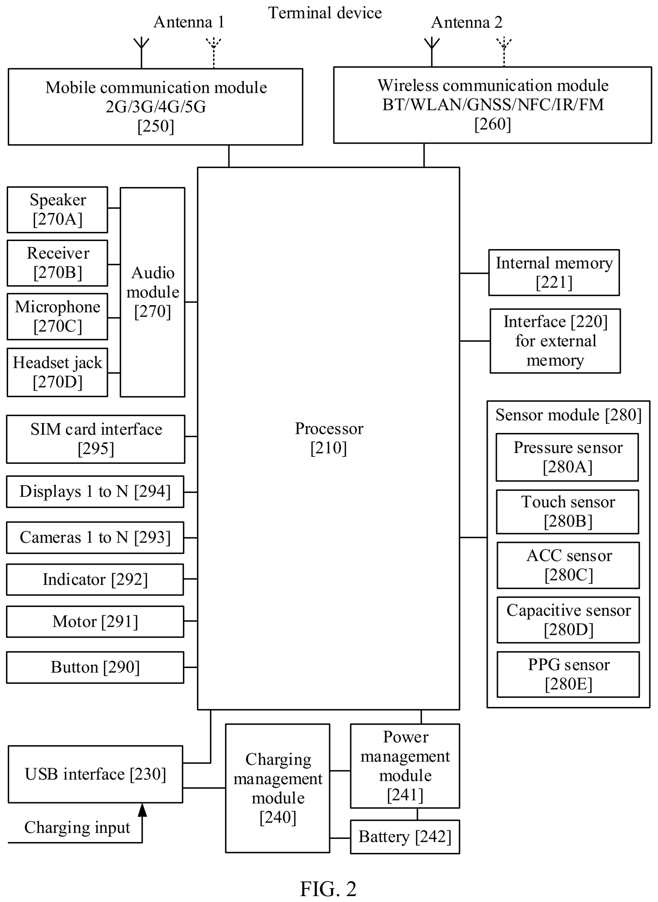

To better understand embodiments of this application, the following describes a hardware structure of the terminal device in embodiments of this application. For example, FIG. 2 is a diagram of a structure of a terminal device according to an embodiment of this application.

The terminal device may include a processor 210, an interface 220 for external memory, an internal memory 221, a universal serial bus (universal serial bus, USB) interface 230, a charging management module 240, a power management module 241, a battery 242, an antenna 1, an antenna 2, a mobile communication module 250, a wireless communication module 260, an audio module 270, a speaker 270A, a receiver 270B, a microphone 270C, a headset jack 270D, a sensor module 280, a button 290, a motor 291, an indicator 292, a camera 293, a display 294, a subscriber identity module (subscriber identification module, SIM) card interface 295, and the like.

Optionally, the sensor module 280 may include a pressure sensor 280A, a touch sensor 280B, an ACC sensor 280C, a capacitive sensor 280D, a PPG sensor 280E, and the like.

The processor 210 may include one or more processing units. For example, the processor 210 may include an application processor (application processor, AP), a modem processor, a graphics processing unit (graphics processing unit, GPU), an image signal processor (image signal processor, ISP), a controller, a video codec, a digital signal processor (digital signal processor, DSP), and the like. Different processing units may be independent components, or may be integrated into one or more processors. A memory may be further disposed in the processor 210, and is configured to store instructions and data.

The USB interface 230 is an interface that conforms to a USB standard specification, and may be specifically a mini USB interface, a micro USB interface, a USB Type-C interface, or the like. The USB interface 230 may be configured to be connected to a charger to charge the terminal device, or may be configured to transmit data between the terminal device and a peripheral device, or may be configured to be connected to a headset for playing audio through a headset. Alternatively, the interface may be configured to be connected to another terminal device, for example, an AR device.

The charging management module 240 is configured to receive a charging input from the charger. The charger may be a wireless charger or a wired charger. When charging the battery 242, the charging management module 240 may further charge the terminal device through the power management module 241.

The power management module 241 is configured to be connected to the battery 242, the charging management module 240, and the processor 210. The power management module 241 receives an input from the battery 242 and/or the charging management module 240, and supplies power to the processor 210, the internal memory 221, an external memory, the camera 293, the display 294, the audio module 270, the wireless communication module 260, and the like. In some embodiments, the power management module 241 may alternatively be disposed in the processor 210. In some other embodiments, the power management module 241 and the charging management module 240 may alternatively be disposed in a same component.

Optionally, in some embodiments, the power management module 241 may also be referred to as a power management unit (power management unit, PMU), and the PWU may be configured to obtain state information of the battery 242, for example, a remaining battery level, a temperature, and the like of the battery 242.

A wireless communication function of the terminal device may be implemented through the antenna 1, the antenna 2, the mobile communication module 250, the wireless communication module 260, the modem processor, the baseband processor, and the like.

The antenna 1 and the antenna 2 are configured to transmit and receive an electromagnetic wave signal. An antenna in the terminal device may be configured to cover one or more communication frequency bands. Different antennas may be further reused, to improve antenna utilization.

The mobile communication module 250 may provide a wireless communication solution that is applied to the terminal device and that includes 2G/3G/4G/5G or the like. The mobile communication module 250 may include at least one filter, a switch, a power amplifier, a low noise amplifier (low noise amplifier, LNA), and the like. The mobile communication module 250 may receive an electromagnetic wave through the antenna 1, perform processing such as filtering or amplification on the received electromagnetic wave, and transmit the processed electromagnetic wave to the modem processor for demodulation. The mobile communication module 250 may further amplify a signal modulated by the modem processor, and convert an amplified signal into an electromagnetic wave for radiation through the antenna 1.

The wireless communication module 260 may provide a wireless communication solution that is applied to the terminal device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), and the like. The wireless communication module 260 may be one or more components integrating at least one communication processing module. The wireless communication module 260 receives an electromagnetic wave through the antenna 2, performs frequency modulation and filtering processing on the electromagnetic wave signal, and sends the processed signal to the processor 210. The wireless communication module 260 may further receive a to-be-sent signal from the processor 210, perform frequency modulation and amplification on the signal, and convert the signal into an electromagnetic wave for radiation through the antenna 2.

In some embodiments, the antenna 1 of the terminal device is coupled to the mobile communication module 250, and the antenna 2 is coupled to the wireless communication module 260, so that the mobile phone can communicate with a network and another device by using a wireless communication technology.

The camera 293 is configured to capture a static image or a video. In some embodiments, the terminal device may include one or N cameras 293, where N is a positive integer greater than 1.

The display 294 is configured to display an image, a video, or the like. The display 294 includes a display panel. In some embodiments, the terminal device may include one or N displays 294, where N is a positive integer greater than 1.

In some embodiments, a touch sensor may be disposed in the display 294 to form a touchscreen. This is not limited in embodiments of this application. It may be understood that, in some embodiments, the terminal device may include the display 294, or may not include the display 294. For example, when the terminal device is a band, the terminal device may include the display 294, or may not include the display 294. When the terminal device is a watch, the terminal device may include the display 294.

The interface 220 for external memory may be configured to be connected to an external storage card, for example, a micro SD card, to extend a storage capability of the terminal device. The external storage card communicates with the processor 210 through the interface 220 for external memory, to implement a data storage function. For example, files such as music and videos are stored in the external storage card.

The internal memory 221 may be configured to store computer-executable program code. The executable program code includes instructions. The internal memory 221 may include a program storage area and a data storage area. The program storage area may store an application required by at least one function (for example, a voice playing function or an image playing function) in an operating system, and the like. The data storage area may store data (for example, audio data and a phone book) and the like created in a process of using the terminal device.

The terminal device may implement an audio function such as music playing or recording through the audio module 270, the speaker 270A, the receiver 270B, the microphone 270C, the headset jack 270D, the application processor, and the like.

The audio module 270 is configured to convert digital audio information into an analog audio signal for output, and is also configured to convert an analog audio input into a digital audio signal. The speaker 270A, also referred to as a "loudspeaker", is configured to convert an audio electrical signal into a sound signal. The terminal device may listen to music or answer a call in a hands-free mode through the speaker 270A. The receiver 270B, also referred to as an "earpiece", is configured to convert an audio electrical signal into a sound signal. When a call is answered or voice information is received by the terminal device, the receiver 270B may be put close to a human ear to listen to a voice. The microphone 270C, also referred to as a "mike" or a "mic", is configured to convert a sound signal into an electrical signal. The headset jack 270D is configured to be connected to a wired headset.

The pressure sensor 280A is configured to sense a pressure signal, and may convert the pressure signal into an electrical signal. In some embodiments, the pressure sensor 280A may be disposed on the display 294. When a touch operation is performed on the display 294, the terminal device may detect intensity of the touch operation through the pressure sensor 280A. The terminal device may also calculate a touch location based on a detection signal of the pressure sensor 280A. There are a plurality of types of pressure sensors 280A, for example, a resistive pressure sensor, an inductive pressure sensor, and a capacitive pressure sensor. This is not limited in embodiments of this application.

The touch sensor 280B may be disposed on the display 294. The touch sensor 280B and the display 294 constitute a touchscreen, which is also referred to as a "touch screen". The touch sensor 280B is configured to detect a touch operation performed on or near the touch sensor. The touch sensor 280B may transfer the detected touch operation to the application processor 210 to determine a type of a touch event. A visual output related to the touch operation may be provided on the display 294. In some other embodiments, the touch sensor 280B may alternatively be disposed on a surface of the display 294, and is at a location different from that of the display 294.

The ACC sensor 280C may be used for magnitudes of accelerations of the terminal device in various directions (usually on three axes). Optionally, when the terminal device is a wearable device, if the user wears the wearable device, and the wearable device moves under driving of the user, the ACC sensor 280C may detect magnitudes of accelerations in all directions, to reflect a motion state of a human body.

The capacitive sensor 280D may be configured to detect capacitance between two electrodes, to implement a specific function.

In some embodiments, the capacitive sensor 280D may be configured to detect capacitance between the human body and the terminal device. The capacitance may reflect whether the human body is in good contact with the terminal device, and may be applied to ECG detection. The human body may be used as an electrode. When the capacitive sensor 280D is disposed on an electrode of the terminal device, the capacitive sensor 280D may detect capacitance between the human body and the electrode. When the capacitance detected by the capacitive sensor 280D is excessively large or excessively small, it indicates that the human body is in poor contact with the electrode. When the capacitance detected by the capacitive sensor 280D is moderate, it indicates that the human body is in good contact with the electrode. Whether the human body is in good contact with the electrode affects detection of an electrical signal by the electrode, and further affects generation of the ECG. Therefore, when the terminal device generates the ECG, refer to the capacitance detected by the capacitive sensor 280D.

The PPG sensor 280E may be configured to detect a heart rate, that is, a quantity of heartbeats per unit time. In some embodiments, the PPG sensor 280E may include an optical sending unit and an optical receiving unit. The optical sending unit may irradiate a light beam into a human body (for example, a blood vessel), the light beam is reflected/refracted in the human body, and reflected/refracted light is received by the optical receiving unit, to obtain an optical signal. Because transmittance of blood changes in a fluctuation process, the emitted/refracted light changes, and an optical signal detected by the PPG sensor 280E also changes. The PPG sensor 280E may convert the optical signal into an electrical signal, to determine a heart rate corresponding to the electrical signal.

The button 290 includes a power button, a volume button, and the like. The button 290 may be a mechanical button, or may be a touch button. The terminal device may receive a button input, and generate a button signal input related to a user setting and function control of the terminal device. The motor 291 may generate a vibration prompt. The indicator 292 may be an indicator light, and may be configured to indicate a charging status and a power change, or may be configured to indicate a message, a missed call, a notification, and the like. A timer 296 may be configured to record time information. For example, when recording a video, the terminal device may record start time and end time of the video, and may record a duration of the recorded video.

It may be understood that, a structure illustrated in embodiments of this application does not constitute a specific limitation on the terminal device. In some other embodiments of this application, the terminal device may include more or fewer components than those shown in the figure, or some components may be combined, or some components may be split, or a different component arrangement may be used. The components shown in the figure may be implemented by hardware, software, or a combination of software and hardware.

In some embodiments, the terminal device may be a wearable device, for example, a smartwatch or a smart band. The wearable device may further include a main body (not shown in FIG. 1) and a wrist strap (not shown in FIG. 1). The wrist strap may be fixedly connected or movably connected to the main body. The wrist strap may be wound around a wrist, an arm, a leg, or another part of a body, to fasten the wearable device to the body of the user. The main body may include a housing and a cover. The housing surrounds the cover. For example, the housing includes a groove provided at the top, the cover is accommodated in the groove, and an edge of the cover is adjacent to and is fastened to the groove of the housing, to form a surface of the main body. A structure formed by the housing and the cover has an internal accommodating space, and may accommodate a combination of one or more components shown and not shown in FIG. 1, to implement various functions of the wearable device. Optionally, the main body further includes an input device. The accommodation space inside the structure formed by the cover and the housing may accommodate a part of the input device, and an exposed part of the input device is easy to be in contact with the user.

The wearable device may perform pairing and establish a wireless connection with a wireless communication module of another terminal device through a wireless communication module of the wearable device, to implement wireless communication with the another terminal device through the wireless connection.

Currently, a method for executing a periodic service by a terminal device may include: After receiving an instruction of a user for enabling the periodic service, the terminal device may periodically control, based on an execution periodicity of the periodic service, a sensor related to the periodic service to collect data.

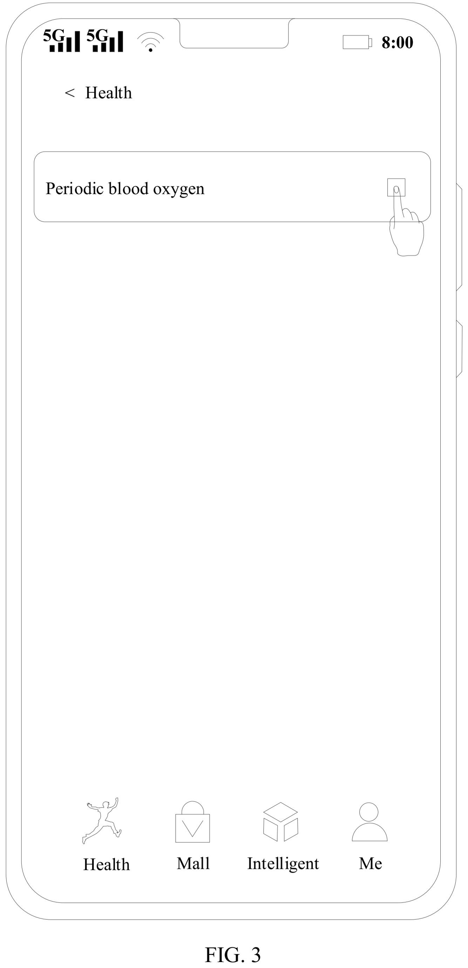

In some scenarios, the terminal device may be a mobile phone. The periodic service being periodic blood oxygen is used as an example. Assuming that a user interface of the terminal device is shown in FIG. 3, and the interface includes an enabling button of the periodic blood oxygen, after the user taps the enabling button, the terminal device may receive an instruction for enabling the periodic blood oxygen, and then periodically controls, through a processor based on an execution periodicity of the periodic blood oxygen, a PPG sensor to collect data; and after collecting the data, the PPG sensor may upload the data to the processor, and then the processor controls a display to display the data.

In some other scenarios, the terminal device may be a wearable device, and the wearable device may communicate with another terminal device (for example, a mobile phone) to jointly execute a periodic service. The periodic service being periodic blood oxygen is used as an example. After the user taps an enabling button on a user interface shown in FIG. 3, the another terminal device may receive an instruction for enabling the periodic blood oxygen, and send indication information to the wearable device, and the indication information indicates the user to enable the periodic blood oxygen. After receiving the instruction, the wearable device may periodically control, through a processor based on an execution periodicity of the periodic blood oxygen, a PPG sensor to collect data; and after collecting the data, the PPG sensor may upload the data to the processor. After receiving the data, the wearable device may send the data to the another terminal device; and then after receiving the data, the another terminal device controls, through a processor of the another terminal device, a display of the another terminal device to display the data.

However, in a use process, it is found that when the terminal device executes a plurality of periodic services, power consumption of the terminal device is high.

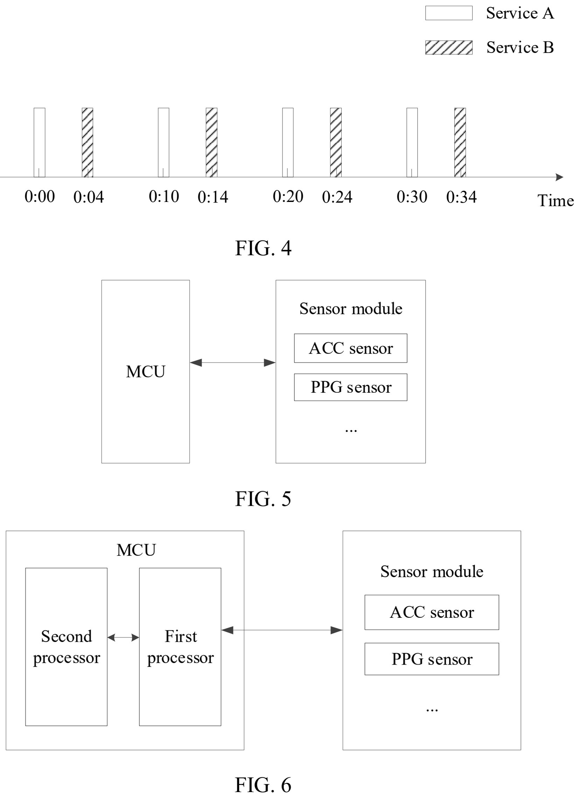

As shown in FIG. 4, it is assumed that both an execution periodicity of a service A and an execution periodicity of a service B are 10 minutes. In this case, assuming that a user taps to enable the service A at 0:00, a terminal device starts to control, at 0:00, a sensor corresponding to the service A to collect and upload data, controls, at 0:10, the sensor corresponding to the service A again to collect and upload data, controls, at 0:20, the sensor corresponding to the service A again to collect and upload data, and so on, until the user disables the service A; and
assuming that the user taps to enable the service B at 0:04, the terminal device starts to control, at 0:04, a sensor corresponding to the service B to collect and upload data, controls, at 0:14, the sensor corresponding to the service B again to collect and upload data, controls, at 0:24, the sensor corresponding to the service B again to collect and upload data, and so on, until the user disables the service B.

Assuming that the sensor corresponding to the service A and the sensor corresponding to the service B include a same sensor, each time the service A or the service B is executed, the terminal device needs to enable the sensor. Consequently, the sensor is enabled for a large quantity of times, and power consumption of the terminal device is high.

Therefore, this application provides a technical solution, to resolve a problem of high power consumption of a terminal device in a conventional technology.

In this application, the terminal device may determine, at a first moment, whether a first service and a second service are in an enabled state, and periodically execute the first service and the second service in the enabled state based on execution periodicities of the services by using the first moment as a start moment.

In the method, the first moment may be preset in advance, and the execution periodicity of the first service and the execution periodicity of the second service may also be preset in advance.

Optionally, when the execution periodicity of the second service is greater than the execution periodicity of the first service, if the first service is not in the enabled state at the first moment, whether the first service is in the enabled state is determined at a second moment.

In some examples, a duration between the second moment and the first moment may be equal to the execution periodicity of the first service. In some other examples, the duration between the second moment and the first moment may alternatively be equal to a greatest common divisor of the execution periodicity of the first service and the execution periodicity of the second service.

Optionally, if the second service is also not in the enabled state at the first moment, whether the second service is in the enabled state is determined at a third moment.

In some examples, a duration between the third moment and the first moment may be equal to the execution periodicity of the second service. In some other examples, the duration between the third moment and the first moment may be equal to the greatest common divisor of the execution periodicity of the first service and the execution periodicity of the second service.

In the method, execution time of the first service and execution time of the second service may overlap at a specific moment, so that a quantity of times of enabling a sensor can be reduced, thereby helping reduce power consumption of the terminal device.

Further, if both the first service and the second service are in the enabled state at a specific moment, a priority of the first service and a priority of the second service may be determined based on a quantity of required sensors, and/or a preset data reporting frequency of a sensor, and/or a preset service running duration, and an execution rank of the first service and an execution rank of the second service are determined based on the priority of the first service and the priority of the second service.

A service that requires a larger quantity of sensors has a higher priority, a service with more preset data reporting frequencies of a sensor has a higher priority, and a service with a shorter preset service running duration has a higher priority.

In the method, the terminal device may first enable a sensor required by a service with a high priority. In this way, when subsequently executing a service with a low priority, the terminal device may directly reuse sensor data of the service with the high priority, so that a quantity of times of reporting the sensor data is reduced, thereby helping reduce power consumption of the terminal device.

In this application, a processor of the terminal device may be a single-core processor. As shown in FIG. 5, the processor of the terminal device may be a control unit (microcontroller unit, MCU) processor. The MCU processor may include an M55 core, and the processor may be connected to a sensor module of the terminal device.

In the system, the sensor module of the terminal device may include an ACC sensor, a PPG sensor, and the like.

In the system, the terminal device may control any one or more sensors in the sensor module through the processor. For example, the processor may control the sensor to collect data and report the data.

Optionally, the processor of the terminal device may alternatively be a dual-core processor. As shown in FIG. 6, the processor of the terminal device may be an MCU processor, and the MCU processor may include a first processor and a second processor.

In the system, the first processor and the second processor may be packaged in a same chip, or may be separately packaged in different chips.

For example, the first processor and the second processor may be respectively an M33 core and an M55 core, the M33 core and the M55 core may be connected through a bus, and the M33 core may be connected to the sensor module.

In the system, the M55 core may communicate with the M33 core, and the M33 core may communicate with any one or more sensors in the sensor module.

For example, the M55 core may send service state information to the M33 core, where the service state information indicates whether a user enables a service. The M33 core may determine execution time of the service, and send the execution time of the service to the M55.

In addition, after determining the execution time of the service, the M55 core may control any one or more sensors in the sensor module through the M33 core. For example, the M55 core may control, through the M33 core, the sensor to collect data and report the data.

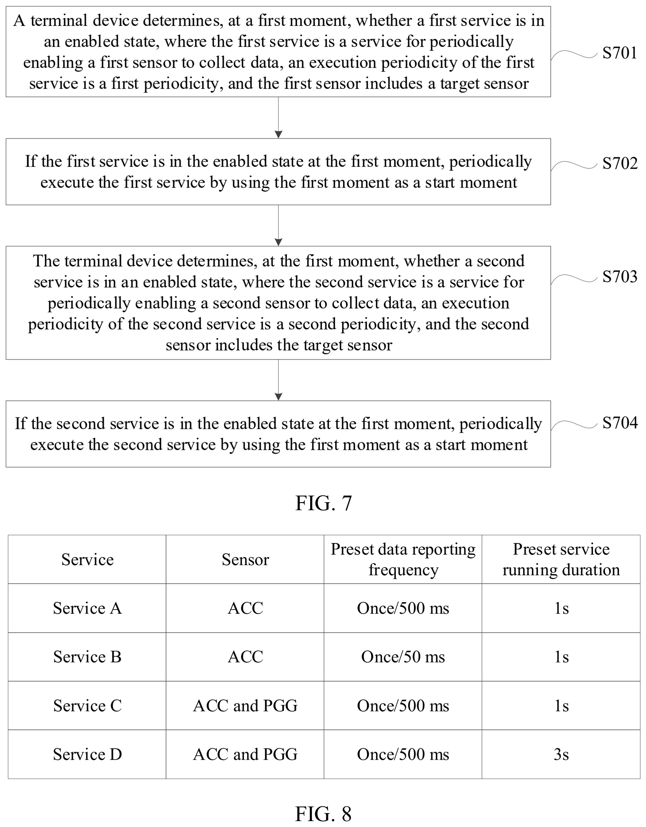

Next, in this application, the solutions of this application are described in detail with reference to FIG. 7 to FIG. 15.

FIG. 7 is a schematic flowchart of a service processing method according to this application. As shown in FIG. 7, the method may include S701 to S704.

S701: A terminal device determines, at a first moment, whether a first service is in an enabled state, where the first service is a service for periodically enabling a first sensor to collect data, an execution periodicity of the first service is a first periodicity, and the first sensor includes a target sensor.

In the method, the terminal device may be the terminal device shown in FIG. 2. A processor of the terminal device may be the single-core processor shown in FIG. 5, or may be the dual-core processor shown in FIG. 6.

In the method, the first moment may be preset in advance. For example, the first moment may be 0:10.

Optionally, the first moment may alternatively be a power-on moment of the terminal device. For example, assuming that the terminal device is powered on at 0:10, the first moment may be 0:10.

Optionally, a duration between the first moment and the power-on moment of the terminal device may alternatively be a preset duration. The preset duration may be preset in advance. For example, assuming that the terminal device is powered on at 0:10, and the preset duration is five minutes, the first moment may be 0:15.

Optionally, the execution periodicity of the first service may also be preset in advance. For example, the execution periodicity of the first service may be 10 minutes.

In the method, the first sensor may be a sensor that needs to be scheduled when the terminal device executes the first service. There may be one or more first sensors. For example, the first sensor may include an ACC sensor.

In a possible implementation, a method for determining, by the terminal device at the first moment, whether the first service is in the enabled state may include: The terminal device first determines, at the first moment, whether a user enables the first service.

In an example, the terminal device may be a mobile phone. The first service being periodic blood oxygen is used as an example. Assuming that a user interface of the terminal device is shown in FIG. 3, and the interface includes an enabling button of the periodic blood oxygen, after the user taps the enabling button, the terminal device may receive an instruction for enabling the periodic blood oxygen, and determine, based on the instruction, that the first service is in the enabled state.

In another example, the terminal device may be a wearable device, and the wearable device may communicate with another terminal device (for example, a mobile phone). The periodic service being periodic blood oxygen is used as an example. After the user taps an enabling button on a user interface shown in FIG. 3, the another terminal device may receive an instruction for enabling the periodic blood oxygen, and send indication information to the wearable device, and the indication information indicates the user to enable the periodic blood oxygen. After receiving the instruction, the wearable device may determine that the first service of the user is in the enabled state.

In the method, when the first service is in the enabled state, the terminal device determines that the first service can be executed, but may not immediately execute the first service. In other words, the first service may be a service that is in the enabled state but has not started to be executed.

S702: If the first service is in the enabled state at the first moment, periodically execute the first service by using the first moment as a start moment.

Assuming that the first moment is 0:10, and the execution periodicity of the first service is 10 minutes, if the first service is in the enabled state at the first moment, the terminal device may execute the first service once at an interval of 10 minutes by using 0:10 as a start moment.

In the method, that the terminal device executes the first service may be understood as that the terminal device sends indication information to the first sensor based on the processor, where the indication information indicates the first sensor to collect data and upload the collected data.

In other words, the terminal device may send the indication information to the first sensor once based on the processor at an interval of 10 minutes. For example, at 0:10, the terminal device may send the indication information to the first sensor once based on the processor. For another example, at 0:20, the terminal device may send the indication information to the first sensor once based on the processor.

In an example, the processor of the terminal device may be a single-core processor. As shown in FIG. 5, assuming that the processor of the terminal device includes an M55 core, the M55 core may send the indication information to the first sensor.

In another example, the processor of the terminal device may be a dual-core processor. As shown in FIG. 6, assuming that the processor of the terminal device includes an M33 core and an M55 core, after determining that the user enables the first service, the M55 core may send information indicating an enabled state/a disabled state of the first service to the M33 core, where the information indicating the enabled state/the disabled state of the first service indicates the user to enable the first service. Optionally, after receiving the indication information, the M33 core may send the indication information to the first sensor.

In the method, each time before executing the first service, the terminal device may further determine, based on the first sensor, whether to execute the first service.

Optionally, a method for determining, by the terminal device based on the first sensor, whether to execute the first service may include: The terminal device may send the indication information to the first sensor through the processor, where the indication information indicates the first sensor to collect and upload data. If the processor receives the data collected by the first sensor or determines that the data collected by the first sensor changes, the terminal device determines to execute the first service.

In an example, the processor of the terminal device may be a single-core processor. As shown in FIG. 5, assuming that the processor of the terminal device includes an M55 core, after determining that the user enables the first service, the M55 core may send the indication information to the first sensor, where the indication information indicates the first sensor to collect and upload data. If the M55 core receives the data collected by the first sensor or determines that the data collected by the first sensor changes, the M55 core may determine to execute the first service.

In another example, the processor of the terminal device may be a dual-core processor. As shown in FIG. 6, assuming that the processor of the terminal device includes an M33 core and an M55 core, after determining that the user enables the first service, the M55 core may send information indicating an enabled state/a disabled state of the first service to the M33 core, where the information indicating the enabled state/the disabled state of the first service indicates the user to enable the first service. Correspondingly, after receiving the indication information, the M33 core may send the indication information to the first sensor, where the indication information indicates the first sensor to collect and upload data. If the M33 core receives the data collected by the first sensor or determines that the data collected by the first sensor changes, the M33 core may determine to execute the first service.

In this example, the method for determining whether to execute the first service may be executed by the M33 core. Because power consumption of the M33 core is lower than power consumption of the M55 core, this helps reduce total power consumption of the terminal device.

Optionally, after the first service starts to be executed, the terminal device may no longer determine whether the first service is in the enabled state.

S703: The terminal device determines, at the first moment, whether a second service is in an enabled state, where the second service is a service for periodically enabling a second sensor to collect data, an execution periodicity of the second service is a second periodicity, and the second sensor includes the target sensor.

Optionally, the execution periodicity of the second service may also be preset in advance. For example, the execution periodicity of the second service may be 30 minutes.

In the method, the second sensor may be a sensor that needs to be scheduled when the terminal device executes the second service. There may be one or more second sensors. For example, the second sensor may include an ACC sensor and a PPG sensor.

In the method, the target sensor may be a sensor shared by the first service and the second service. Assuming that the first sensor includes the ACC sensor, and the second sensor includes the ACC sensor and the PPG sensor, the target sensor may be the ACC sensor.

For a method for determining, by the terminal device, whether the second service is in the enabled state, refer to the method for determining, by the terminal device, whether the first service is in the enabled state in S701. Details are not described herein again.

S704: If the second service is in the enabled state at the first moment, periodically execute the second service by using the first moment as a start moment.

Assuming that the first moment is 0:10, and the execution periodicity of the second service is 30 minutes, if the second service is in the enabled state at the first moment, the terminal device may execute the second service once at an interval of 30 minutes by using 0:10 as a start moment.

In the method, that the terminal device executes the second service may be understood as that the terminal device sends indication information to the second sensor based on the processor, where the indication information indicates the second sensor to collect data and upload the collected data.

In other words, the terminal device may send the indication information to the second sensor once based on the processor at an interval of 30 minutes. For example, at 0:10, the terminal device may send the indication information to the second sensor once based on the processor. For another example, at 0:40, the terminal device may send the indication information to the second sensor once based on the processor.

For a method for sending, by the terminal device, the indication information to the second sensor based on the processor, refer to S702. Details are not described herein again.

Optionally, before subsequently executing the second service, the terminal device may further determine, based on the second sensor, whether to execute the second service.

For a method for determining, by the terminal device based on the second sensor, whether to execute the second service, refer to the method for determining, by the terminal device based on the first sensor, whether to execute the first service in S702. Details are not described herein again.

In the method, execution time of the first service and execution time of the second service may overlap at a specific moment. A duration between the specific moment and the first moment may be a common multiple of the first periodicity and the second periodicity.

In this way, the target sensor needs to be enabled only once at the specific moment, so that a quantity of times of enabling the target sensor can be reduced, thereby helping reduce power consumption of the terminal device.

In some possible implementations, when the second periodicity is greater than the first periodicity, if the first service is not in the enabled state at the first moment, the terminal device determines, at a second moment, whether the first service is in the enabled state, where a duration between the second moment and the first moment may be equal to a first duration, and the first duration may be equal to a greatest common divisor of a duration of the first periodicity and a duration of the second periodicity.

In an example, assuming that the first periodicity is 10 minutes, and the second periodicity is 30 minutes, the first duration may be 10 minutes. Assuming that the first moment is 0:10, the second moment may be 0:20.

In this example, the greatest common divisor of the duration of the first periodicity and the duration of the second periodicity is equal to the duration of the first periodicity, and the duration between the second moment and the first moment may be equal to the duration of the first periodicity. In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the second moment and the first moment may be equal to the duration of the first periodicity. In this way, a time interval between two consecutive times of determining, by the terminal device, whether the first service is in the enabled state may be equal to the duration of the first periodicity. This helps ensure a value output rate of the first service.

In another example, assuming that the first periodicity is 10 minutes, and the second periodicity is 15 minutes, the first duration may be five minutes. Assuming that the first moment is 0:10, the second moment may be 0:15.

In this example, the duration between the second moment and the first moment may be equal to the greatest common divisor of the duration of the first periodicity and the duration of the second periodicity. In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the second moment and the first moment may be less than the duration of the first periodicity. In this way, a time interval between two consecutive times of determining, by the terminal device, whether the first service is in the enabled state may be less than the duration of the first periodicity. This helps ensure a value output rate of the first service.

Optionally, in some other examples, assuming that the first periodicity is five minutes, the second periodicity is six minutes, and the first moment is 0:10, the second moment may be 0:15, and the duration between the second moment and the first moment may be equal to the duration of the first periodicity.

In this example, because the duration of the first periodicity and the duration of the second periodicity have only one common divisor, and the common divisor is 1, the duration between the second moment and the first moment may be equal to the duration of the first periodicity. In this way, a case in which whether to enable the first service is determined for a plurality of times based on the greatest common divisor of the duration of the first periodicity and the duration of the second periodicity can be avoided. This helps reduce power consumption of the terminal device.

In addition, in this example, the execution time of the first service and the execution time of the second service may alternatively overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the second moment and the first moment may be equal to the duration of the first periodicity. In this way, a time interval between two consecutive times of determining, by the terminal device, whether the first service is in the enabled state may be equal to the duration of the first periodicity. This helps ensure a value output rate of the first service.

Optionally, in some examples, if the terminal device starts to periodically execute the second service at the first moment, the terminal device may not determine, at the second moment, whether to execute the second service.

In some possible implementations, if the second service is not in the enabled state at the first moment, whether the second service is in the enabled state is determined at a third moment; and if the second service is in the enabled state at the third moment, the second service is periodically executed by using the third moment as a start moment.

Optionally, a duration between the third moment and the first moment may alternatively be equal to the first duration.

In some examples, assuming that the first periodicity is 10 minutes, and the second periodicity is 30 minutes, the first duration may be 10 minutes. Assuming that the first moment is 0:10, the second moment may be 0:20, and the third moment may also be 0:20.

In this example, the third moment and the second moment may be a same moment. In this way, when neither the first service nor the second service is in the enabled state at the first moment, whether the first service and the second service are in the enabled state may be determined at the second moment (or the third moment). When it is determined that the first service and the second service are in the enabled state at the second moment (or the third moment), each service may be periodically executed based on an execution periodicity of each service.

In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the third moment and the first moment may be equal to the duration of the first periodicity, and the duration of the first periodicity is less than the duration of the second periodicity. In this way, a time interval between two consecutive times of determining, by the terminal device, whether the second service is in the enabled state may be less than the duration of the second periodicity. This helps ensure a value output rate of the second service.

In some other examples, assuming that the first periodicity is 10 minutes, and the second periodicity is 15 minutes, the first duration may be five minutes. Assuming that the first moment is 0:10, the second moment may be 0:15, and the third moment may be 0:15.

In this example, the third moment and the second moment may be a same moment. In this way, when neither the first service nor the second service is in the enabled state at the first moment, whether the first service and the second service are in the enabled state may be determined at the second moment (or the third moment). When it is determined that the first service and the second service are in the enabled state at the second moment (or the third moment), each service may be periodically executed based on an execution periodicity of each service.

In this way, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the third moment and the first moment may be less than the duration of the first periodicity, and the duration of the first periodicity is less than the duration of the second periodicity. In this way, a time interval between two consecutive times of determining, by the terminal device, whether the second service is in the enabled state may be less than the duration of the second periodicity. This helps ensure a value output rate of the second service.

Optionally, a duration between the third moment and the first moment may alternatively be equal to the duration of the second periodicity.

For example, assuming that the first periodicity is five minutes, the second periodicity is six minutes, and the first moment is 0:10, the second moment may be 0:15, and the third moment may be 0:16.

In this example, because the duration of the first periodicity and the duration of the second periodicity have only one common divisor, and the common divisor is 1, the duration between the third moment and the first moment may be equal to the duration of the second periodicity. In this way, a case in which whether to enable the second service is determined for a plurality of times based on the greatest common divisor of the duration of the first periodicity and the duration of the second periodicity can be avoided. This helps reduce power consumption of the terminal device.

In addition, in this example, the execution time of the first service and the execution time of the second service may alternatively overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the duration between the third moment and the first moment may be equal to the duration of the second periodicity. In this way, a time interval between two consecutive times of determining, by the terminal device, whether the second service is in the enabled state may be equal to the duration of the second periodicity. This helps ensure a value output rate of the second service.

Optionally, in some examples, if the terminal device starts to periodically execute the first service at the first moment, the terminal device may not determine, at the third moment, whether to execute the first service.

In some possible implementations, if the second service is not in the enabled state at the third moment and it is determined that the second service is not in the enabled state at each of M moments, whether the second service is in the enabled state is determined at a fourth moment, where an interval between the fourth moment and the third moment is a duration of one second periodicity, M is a positive integer, a duration of an interval between an m^{th} moment in the M moments and the first moment is equal to m first durations, m is a positive integer and ranges from 1 to M, an M^{th} moment in the M moments is before the fourth moment, an (M+1)^{th} moment is at the fourth moment or after the fourth moment, and a duration of an interval between the (M+1)^{th} moment and the first moment is equal to (M+1) first durations.

In an example, assuming that the execution periodicity of the first service is 10 minutes, and the execution periodicity of the second service is 30 minutes, the first duration may be 10 minutes. Assuming that the first moment is 0:10, if the terminal device determines, at 0:10, that the second service is not in the enabled state, the terminal device re-determines, at 0:20, whether the second service is in the enabled state; if the terminal device determines, at 0:20, that the second service is not in the enabled state, the terminal device re-determines, at 0:30, whether the second service is in the enabled state; if the terminal device determines, at 0:30, that the second service is not in the enabled state, the terminal device re-determines, at 0:40, whether the second service is in the enabled state; and if the terminal device determines, at 0:40, that the second service is not in the enabled state, the terminal device re-determines, at 0:50, whether the second service is in the enabled state.

In this example, 0:20 may be the third moment, the M moments may include three moments: 0:20, 0:30, and 0:40, the fourth moment may include 0:50, and the fourth moment is the (M+1)^{th} moment.

In another example, assuming that the execution periodicity of the first service is 10 minutes, and the execution periodicity of the second service is 15 minutes, the first duration may be five minutes. Assuming that the first moment is 0:10, if the terminal device determines, at 0:10, that the second service is not in the enabled state, the terminal device re-determines, at 0:15, whether the second service is in the enabled state; if the terminal device determines, at 0:15, that the second service is not in the enabled state, the terminal device re-determines, at 0:20, whether the second service is in the enabled state; if the terminal device determines, at 0:20, that the second service is not in the enabled state, the terminal device re-determines, at 0:25, whether the second service is in the enabled state; and if the terminal device determines, at 0:25, that the second service is not in the enabled state, the terminal device re-determines, at 0:30, whether the second service is in the enabled state.

In this example, 0:15 may be the third moment, the M moments may include three moments: 0:15, 0:20, and 0:25, the fourth moment may include 0:30, and the fourth moment is the (M+1)^{th} moment.

In the method, the execution time of the first service and the execution time of the second service may overlap at the specific moment, so that the quantity of times of enabling the target sensor can be reduced, thereby helping reduce power consumption of the terminal device. A duration between the specific moment and the second moment may be a common multiple of the first periodicity and the second periodicity.

In addition, the first duration may be less than or equal to the duration of the first periodicity, and the duration of the first periodicity is less than the duration of the second periodicity. In this way, a time interval between two consecutive times of determining, by the terminal device, whether the second service is in the enabled state may be less than the duration of the second periodicity. This helps ensure a value output rate of the second service.

Optionally, in some embodiments, the terminal device may alternatively not need to determine, at a specific moment (for example, the first moment or the third moment described above), whether the second service is at an enabling moment. For example, the terminal device may set a detection periodicity, and all services may determine, based on the detection periodicity, whether all the services are at the enabling moment.

In the technical solution of this application, if both the first service and the second service are in the enabled state at the target moment, a priority of the first service and a priority of the second service may be determined based on at least one of a first condition, a second condition, and a third condition, and then an execution rank of the first service and an execution rank of the second service are determined in descending sequence of the priority of the first service and the priority of the second service.

The first condition may be that a service that requires a larger quantity of sensors has a higher priority. The second condition may be that a service with more preset data reporting frequencies of a sensor has a higher priority. The third condition may be that a service with a shorter preset service running duration has a higher priority.

Optionally, a sensor required by each service may be preset in advance. For example, a sensor required by the first service is the first sensor, and a sensor required by the second service is the second sensor.

Optionally, a preset reporting frequency, of a sensor, preset for each service may alternatively be preset in advance.

Optionally, a preset service running duration of each service may alternatively be preset in advance.

In a possible implementation, that the terminal device determines the execution rank of the first service and the execution rank of the second service in descending sequence of the priority of the first service and the priority of the second service may include: if the priority of the first service is higher than the priority of the second service, determining to enable the first sensor at the target moment, and enable a sensor in the second sensor other than the target sensor after the target moment; or if the priority of the first service is lower than the priority of the second service, determining to enable the second sensor at the target moment, and enable a sensor in the first sensor other than the target sensor after the target moment.

In a first example, assuming that a quantity of first sensors is greater than a quantity of second sensors, and when a preset reporting frequency of a sensor and a preset service running duration of the first service is the same as a preset reporting frequency of a sensor and a preset service running duration of the second service, the priority of the first service is higher than the priority of the second service, and the execution rank of the first service is higher than the execution rank of the second service.

For example, assuming that the first sensor includes an ACC sensor and a PPG sensor, the second sensor includes an ACC sensor, and a quantity of first sensors is greater than a quantity of second sensors, the priority of the first service is higher than the priority of the second service, and the execution rank of the first service is higher than the execution rank of the second service.

In this example, the target sensor may be the ACC sensor. The terminal device may first enable the first sensor, and then enable a sensor in the second sensor other than the target sensor. In this way, a quantity of times of enabling the target sensor can be reduced, thereby helping reduce power consumption of the terminal device.

In a second example, assuming that when a preset reporting frequency of a sensor of the first service is greater than a preset reporting frequency of a sensor of the second service, a quantity of first sensors is the same as a quantity of second sensors, and a preset service running duration of the first service is also the same as a preset service running duration of the second service, the priority of the first service is higher than the priority of the second service, and the execution rank of the first service is higher than the execution rank of the second service.

For example, assuming that a preset reporting frequency of a sensor of the first service is reporting once per 50 milliseconds, and a preset reporting frequency of a sensor of the second service is reporting once per 500 milliseconds, the priority of the first service is higher than the priority of the second service, and the execution rank of the first service is higher than the execution rank of the second service.

In this example, the terminal device may first enable the first sensor, and then enable a sensor in the second sensor other than the target sensor. In this way, a quantity of times of enabling the target sensor can be reduced, thereby helping reduce power consumption of the terminal device.

In a third example, assuming that when a preset service running duration of the first service is less than a preset service running duration of the second service, a quantity of first sensors is the same as a quantity of second sensors, and a preset reporting frequency of a sensor of the first service is also the same as a preset reporting frequency of a sensor of the second service, the priority of the first service is higher than the priority of the second service, and the execution rank of the first service is higher than the execution rank of the second service.

For example, assuming that a preset service running duration of the first service is 1 second, and a preset service running duration of the second service is 3 seconds, the priority of the first service is higher than the priority of the second service, and the execution rank of the first service is higher than the execution rank of the second service.

In this example, the terminal device may first enable the first sensor, and then enable a sensor in the second sensor other than the target sensor. In this way, a quantity of times of enabling the target sensor can be reduced, thereby helping reduce power consumption of the terminal device.

Optionally, if at least two parameters of the quantity of sensors in the first service and the second service, the preset data reporting frequency of the sensor, and the preset service running duration are different, the priority of the first service and the priority of the second service may be determined based on determining priorities of the first condition, the second condition, and the third condition.

The determining priority of the first condition may be higher than the determining priority of the second condition, and the determining priority of the second condition may be higher than the determining priority of the third condition.

Optionally, after determining the execution rank of the first service and the execution rank of the second service in descending sequence of the priority of the first service and the priority of the second service, the terminal device may further determine a target data reporting frequency based on a preset data reporting frequency of the first service and a preset data reporting frequency of the second service, where the target data reporting frequency is equal to a greatest common divisor of the preset data reporting frequency of the first service and the preset data reporting frequency of the second service; and then determine the target data reporting frequency as a data reporting frequency of the target sensor after the target moment.

In an example, assuming that a preset reporting frequency of a sensor of the first service is reporting once per 50 milliseconds, and a preset reporting frequency of a sensor of the second service is reporting once per 500 milliseconds, the greatest common divisor of the preset data reporting frequency of the first service and the preset data reporting frequency of the second service is reporting once per 50 milliseconds, and the target data reporting frequency may be reporting once per 50 milliseconds.

In another example, assuming that a preset reporting frequency of a sensor of the first service is reporting once per 50 milliseconds, and a preset reporting frequency of a sensor of the second service is reporting once per 60 milliseconds, the greatest common divisor of the preset data reporting frequency of the first service and the preset data reporting frequency of the second service is reporting once per 10 milliseconds, and the target data reporting frequency may be reporting once per 10 milliseconds.

In the method, the data reporting frequency of the target sensor after the target moment may be the target data reporting frequency. In this way, it can be ensured that the data reporting frequency of the target sensor after the target moment can meet both a data reporting frequency requirement of the first service and a data reporting frequency requirement of the second service.

Further, the terminal device may further determine a target service from the first service and the second service, where the target service is a service with a shorter preset service running duration in the first service and the second service; if a preset service running duration of the target service is shorter than a preset service running duration of another service that is not the target service in the first service and the second service, determine an end moment of the target service based on the preset service running duration of the target service, and an interval between the end moment and the target moment is equal to a preset service duration of the target service; and determine a preset data reporting frequency of the another service as a data reporting frequency of the target sensor after the end moment.

For example, assuming that the preset service running duration of the first service is 1 second, the preset service running duration of the second service is 3 seconds, and the target moment is 0:30, the terminal device determines that the data reporting frequency of the target sensor after 0:30 is the target data reporting frequency, and determines that the data reporting frequency of the target sensor after 0:31 is the preset data reporting frequency of the second service.

In this example, it can be avoided that the target sensor collects and reports some redundant data when the target sensor still performs reporting at a high data reporting frequency after running of the first service is completed, thereby helping further reduce power consumption of the terminal device.

Optionally, after determining the data reporting frequency of the target sensor, the terminal device may send indication information to the target sensor based on the processor, where the indication information indicates the target sensor to perform reporting based on the data reporting frequency determined by the terminal device.

For a method for sending, by the terminal device, the indication information to the target sensor based on the processor, refer to the step of sending, by the terminal device, the indication information to the first sensor based on the processor in S701. Details are not described herein again.

It may be understood that the foregoing embodiment is described by using the first service and the second service as an example. Optionally, when more than two services are included, the foregoing method is also applicable.

For example, assuming that at 0:00, the terminal device determines that a service A, a service B, a service C, and a service D are all in an enabled state, and the terminal device determines that the service A, the service B, the service C, and the service D need to be executed.

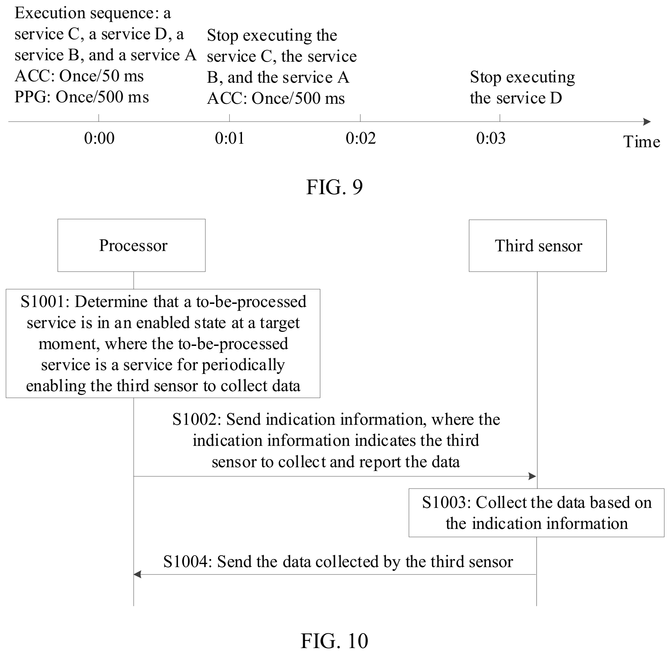

As shown in FIG. 8, it is assumed that a sensor required by the service A is an ACC sensor, a preset data reporting frequency of the sensor of the service A is reporting once per 500 milliseconds, and a preset service running duration of the service A is 1 second; a sensor required by the service B is an ACC sensor, a preset data reporting frequency of the sensor of the service B is reporting once per 50 milliseconds, and a preset service running duration of the service B is 1 second; sensors required by the service C are an ACC sensor and a PPG sensor, a preset data reporting frequency of the sensors of the service C is reporting once per 500 milliseconds, and a preset service running duration of the service C is 1 second; and sensors required by the service D are an ACC sensor and a PPG sensor, a preset data reporting frequency of the sensors of the service D is reporting once per 500 milliseconds, and a preset service running duration of the service C is 3 seconds,
the terminal device may determine, based on the parameter information such as the quantity of sensors required by each of the four services, the preset data reporting frequency of the sensor, and the preset service running duration, that a descending sequence of priorities is: the service C, the service D, the service B, and the service A, and the terminal device may determine, based on the priority sequence, that an execution sequence is: the service C, the service D, the service B, and the service A, and determine scheduling of the service A, the service B, the service C, and the service D, as shown in FIG. 9.

In this example, at 0:00, the terminal device executes the service A, the service B, the service C, and the service D based on the execution sequence, determines that the data reporting frequency of the ACC sensor is reporting once per 50 milliseconds, and determines that the data reporting frequency of the PPG is reporting once per 500 milliseconds;
at 0:01, the terminal device stops executing the service A, the service B, and the service C, and determines that the data reporting frequency of the ACC sensor is reporting once per 500 milliseconds; and
at 0:03, the terminal device stops executing the service D.

Next, a method for obtaining sensor data by the terminal device is described in detail in this application. FIG. 10 is a schematic flowchart of a service processing method for a terminal device according to another embodiment of this application.

In the method, a processor of the terminal device may be the single-core processor shown in FIG. 5.

S1001: The processor of the terminal device determines that a to-be-processed service is in an enabled state at a target moment, where the to-be-processed service is a service for periodically enabling a third sensor to collect data.

For example, the to-be-processed service may include a first service and/or a second service. The third sensor may be a sensor required by the to-be-processed service.

In an example, assuming that the to-be-processed service includes the first service, the third sensor may be a first sensor.

In another example, assuming that the to-be-processed service includes the second service, the third sensor may be a second sensor.

In still another example, assuming that the to-be-processed service includes the first service and the second service, the third sensor may include a first sensor and a second sensor, and both the first sensor and the second sensor include a target sensor.

For a method for determining, by the terminal device, that the to-be-processed service is in the enabled state at the target moment, refer to the step of determining, by the terminal device, that the first service is in the enabled state in S701. Details are not described herein again.

S1002: When determining to execute the to-be-processed service, the processor of the terminal device sends indication information to the third sensor, where the indication information indicates the third sensor to collect and report the data. Correspondingly, the third sensor receives the indication information.

For a method for determining, by the processor of the terminal device, to execute the to-be-processed service, refer to the step of determining, by the terminal device, to execute the first service in S702. Details are not described herein again.

Optionally, when the to-be-processed service includes the first service and the second service, the terminal device may send the indication information to the first sensor and the second sensor based on a priority of the first service and a priority of the second service.

For a method for determining, by the processor of the terminal device, the priority of the first service and the priority of the second service, refer to the foregoing embodiments. Details are not described herein again.

Optionally, the method for sending, by the processor of the terminal device, the indication information to the first sensor and the second sensor based on the priority of the first service and the priority of the second service may include: If the priority of the first service is higher than the priority of the second service, the processor sends first indication information to the first sensor, where the first indication information indicates the first sensor to collect first data and upload the first data at the target moment; and the processor sends second indication information to a sensor in the second sensor other than the target sensor, where the second indication information indicates the another sensor to collect second data and upload the second data after the target moment; or if the priority of the first service is lower than the priority of the second service, the first processor sends third indication information to the second sensor, where the third indication information indicates the second sensor to collect second data and upload the second data at the target moment; and the processor sends fourth indication information to a sensor in the first sensor other than the target sensor, where the fourth indication information indicates the another sensor to collect first data and upload the first data after the target moment.

Further, the processor may further send fifth indication information to the target sensor, where the fifth indication information indicates the target sensor to report data based on a target data reporting frequency after the target moment, and the target data reporting frequency is equal to a greatest common divisor of a preset data reporting frequency of the first service and a preset data reporting frequency of the second service.

Further, the processor may further send sixth indication information to the target sensor, where the sixth indication information indicates the target sensor to report data based on a preset data reporting frequency of another service after an end moment of the to-be-processed service, the to-be-processed service is a service with a shorter preset service running duration in the first service and the second service, and the another service is a service other than the to-be-processed service in the first service and the second service.

S1003: The third sensor collects the data based on the indication information.

S1004: The third sensor sends, to the processor, the data collected by the third sensor. Correspondingly, the processor receives the data collected by the third sensor.

In the method, the processor of the terminal device may be a single-core processor. A method for determining, by the terminal device, whether to enable the to-be-processed service and a method for determining, by the terminal device, to execute the to-be-processed service are both performed by the processor. When the to-be-processed service includes the first service and the second service, execution time of the first service and execution time of the second service may overlap in specific time. In this way, not only power consumption of the terminal device can be reduced, but also response time of executing the to-be-processed service by the terminal device can be reduced, thereby improving a response speed of the to-be-processed service.

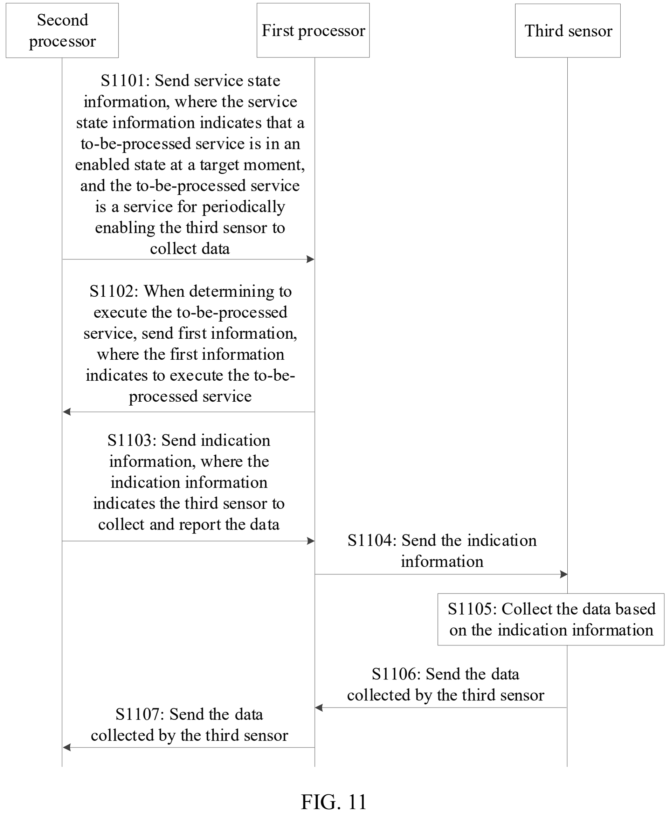

Optionally, FIG. 11 is a schematic flowchart of a service processing method for a terminal device according to still another embodiment of this application.

In the method, a processor of the terminal device may be the dual-core processor shown in FIG. 6. In this example, the processor of the terminal device may include a first processor and a second processor. The first processor may be the M33 core shown in FIG. 6, and the second processor may be the M55 core shown in FIG. 6.

S1101: The first processor receives service state information from the second processor, where the service state information indicates that a to-be-processed service is in an enabled state at a target moment, and the to-be-processed service is a service for periodically enabling a third sensor to collect data.

For example, the to-be-processed service may include a first service and/or a second service. The third sensor may be a sensor required by the to-be-processed service.

In an example, assuming that the to-be-processed service includes the first service, the third sensor may be a first sensor.

In another example, assuming that the to-be-processed service includes the second service, the third sensor may be a second sensor.

In still another example, assuming that the to-be-processed service includes the first service and the second service, the third sensor may include a first sensor and a second sensor, and both the first sensor and the second sensor include a target sensor.

In the method, for a method for determining, by the second processor, that the first service and the second service are in the enabled state at the target moment, refer to the step of determining, by the terminal device, that the first service is in the enabled state in S701. Details are not described herein again.

S1102: When determining to execute the to-be-processed service, the first processor sends first information to the second processor, where the first information indicates to execute the to-be-processed service. Correspondingly, the second processor receives the first information.

In the method, for a method for determining, by the first processor, to execute the to-be-processed service, refer to the step of determining, by the terminal device, to execute the first service in S702. Details are not described herein again.

S1103: The second processor sends indication information to the first processor, where the indication information indicates the third sensor to collect and report the data. Correspondingly, the first processor receives the indication information.

S1104: The first processor sends the indication information to the third sensor. Correspondingly, the third sensor receives the indication information.

Optionally, when the to-be-processed service includes the first service and the second service, the second processor of the terminal device may send the indication information to the first processor based on a priority of the first service and a priority of the second service.

For a method for determining, by the second processor of the terminal device, the priority of the first service and the priority of the second service, refer to the foregoing embodiments. Details are not described herein again.

Optionally, the method for sending, by the second processor of the terminal device, the indication information to the first processor based on the priority of the first service and the priority of the second service may include:

If the priority of the first service is higher than the priority of the second service, the second processor sends first indication information to the first processor, where the first indication information indicates the first sensor to collect first data and upload the first data at the target moment. Correspondingly, after receiving the first indication information, the first processor may send the first indication information to the first sensor. The second processor sends second indication information to the first processor, where the second indication information indicates a sensor in the second sensor other than the target sensor to collect second data and upload the second data after the target moment. Correspondingly, after receiving the second indication information, the first processor may send the second indication information to the another sensor.

Alternatively, if the priority of the first service is lower than the priority of the second service, the second processor sends third indication information to the first processor, where the third indication information indicates the second sensor to collect second data and upload the second data at the target moment. Correspondingly, after receiving the third indication information, the first processor may send the third indication information to the second sensor. The second processor sends fourth indication information to the first processor, where the fourth indication information indicates the another sensor to collect first data and upload the first data after the target moment. Correspondingly, after receiving the fourth indication information, the first processor may send the fourth indication information to the another sensor.

Further, the second processor may further send fifth indication information to the first processor, where the fifth indication information indicates the target sensor to report data based on a target data reporting frequency after the target moment, and the target data reporting frequency is equal to a greatest common divisor of a preset data reporting frequency of the first service and a preset data reporting frequency of the second service. Correspondingly, after receiving the fifth indication information, the first processor may send the fifth indication information to the target sensor.

Further, the second processor may further send sixth indication information to the first processor, where the sixth indication information indicates the target sensor to report data based on a preset data reporting frequency of another service after an end moment of the to-be-processed service, the to-be-processed service is a service with a shorter preset service running duration in the first service and the second service, and the another service is a service in the first service and the second service other than the to-be-processed service. Correspondingly, after receiving the sixth indication information, the first processor may send the sixth indication information to the target sensor.

S1105: The third sensor collects the data based on the indication information.

S1106: The third sensor sends, to the first processor, the data collected by the third sensor. Correspondingly, the first processor receives the data collected by the third sensor.

S1107: The first processor sends, to the second processor, the data collected by the third sensor. Correspondingly, the second processor receives the data collected by the third sensor.

In the method, the processor of the terminal device may be a dual-core processor. A method for determining, by the terminal device, whether to enable the to-be-processed service and a method for determining, by the terminal device, to execute the to-be-processed service are both performed by the first processor. Because power consumption of the first processor is lower than power consumption of the second processor, power consumption of the terminal device can be further reduced.

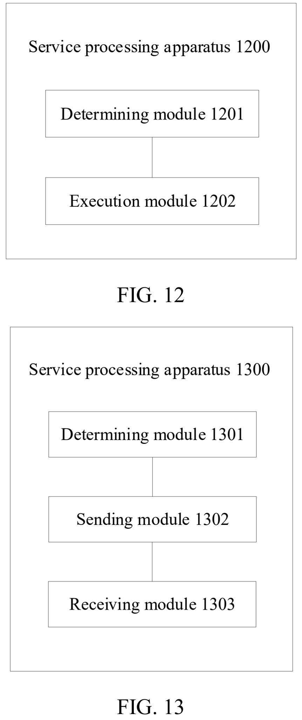

FIG. 12 is a diagram of a structure of a service processing apparatus according to an embodiment of this application. As shown in FIG. 12, the service processing apparatus 1200 may include a determining module 1201 and an execution module 1202.

In an example, the service processing apparatus 1200 may be configured to implement the service processing method in the embodiment shown in FIG. 7. The determining module 1201 may be configured to perform S701 and S703, and the execution module 1202 may be configured to perform S702 and S704.

Optionally, the service processing apparatus 1200 may be used in a terminal device. For example, the service processing apparatus 1200 may be used in a processor of the terminal device. The processor of the terminal device may be the processor shown in FIG. 5 or FIG. 6.

FIG. 13 is a diagram of a structure of a service processing apparatus according to an embodiment of this application. As shown in FIG. 13, the service processing apparatus 1300 may include a determining module 1301, a sending module 1302, and a receiving module 1303.

In an example, the service processing apparatus 1300 may be configured to implement the service processing method in the embodiment shown in FIG. 10. The determining module 1301 may be configured to perform S1001, the sending module 1302 may be configured to perform S1002, and the receiving module 1303 may be configured to perform S1004.

Optionally, the service processing apparatus 1300 may be used in the processor of the terminal device shown in FIG. 5.

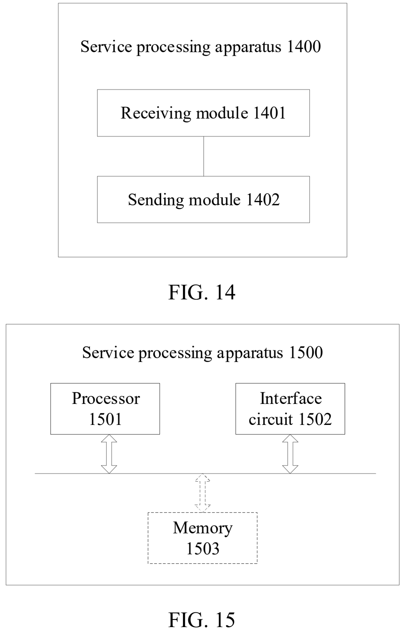

FIG. 14 is a diagram of a structure of a service processing apparatus according to an embodiment of this application. As shown in FIG. 14, the service processing apparatus 1400 may include a receiving module 1401 and a sending module 1402.

In an example, the service processing apparatus 1400 may be configured to implement the service processing method in the embodiment shown in FIG. 14. The receiving module 1401 may be configured to perform S1101, S1103, and S1106, and the sending module 1402 may be configured to perform S1102, S1104, and S1107.

Optionally, the service processing apparatus 1400 may be used in the first processor of the terminal device shown in FIG. 6.

FIG. 15 is a diagram of a structure of a service processing apparatus according to still another embodiment of this application. As shown in FIG. 15, the service processing apparatus 1500 includes a processor 1501 and an interface circuit 1502. The processor 1501 and the interface circuit 1502 are coupled to each other. It may be understood that the interface circuit 1502 may be a transceiver or an input/output interface. Optionally, the service processing apparatus 1500 may further include a memory 1503, configured to store instructions executed by the processor 1501, or store input data required by the processor 1501 to run instructions, or store data generated after the processor 1501 runs instructions.

In a first example, the processor 1501 may be configured to implement functions of the determining module 1201 and the execution module 1202.

In a second example, the processor 1501 may be configured to implement a function of the determining module 1301, and the interface circuit 1502 is configured to implement functions of the sending module 1302 and the receiving module 1303.

In a second example, the interface circuit 1502 is configured to implement functions of the receiving module 1401 and the sending module 1402.

The service processing apparatus 1500 may be a terminal device, or may be a chip used in the terminal device.

The method steps in embodiments of this application may be implemented in a hardware manner, or may be implemented in a manner of executing software instructions by the processor. The software instructions may include a corresponding software module. The software module may be stored in a random access memory, a flash memory, a read-only memory, a programmable read-only memory, an erasable programmable read-only memory, an electrically erasable programmable read-only memory, a register, a hard disk, a removable hard disk, a CD-ROM, or any other form of storage medium well-known in the art. For example, a storage medium is coupled to a processor, so that the processor can read information from the storage medium and write information into the storage medium. Certainly, the storage medium may be a component of the processor. The processor and the storage medium may be disposed in an ASIC. In addition, the ASIC may be located in a network device or a terminal device. Certainly, the processor and the storage medium may alternatively exist as discrete components in a network device or a terminal device.

All or some of the foregoing embodiments may be implemented by software, hardware, firmware, or any combination thereof. When the software is used to implement embodiments, all or a part of embodiments may be implemented in a form of a computer program product. The computer program product includes one or more computer programs or instructions. When the computer programs or instructions are loaded and executed on a computer, the procedures or functions in embodiments of this application are all or partially executed. The computer may be a general-purpose computer, a dedicated computer, a computer network, a network device, user equipment, or another programmable apparatus. The computer program or instructions may be stored in a computer-readable storage medium, or may be transmitted from a computer-readable storage medium to another computer-readable storage medium. For example, the computer program or instructions may be transmitted from a website, computer, server, or data center to another website, computer, server, or data center in a wired or wireless manner. The computer-readable storage medium may be any usable medium that can be accessed by the computer, or a data storage device, for example, a server or a data center, integrating one or more usable media. The usable medium may be a magnetic medium, for example, a floppy disk, a hard disk, or a magnetic tape; or may be an optical medium, for example, a digital video disc; or may be a semiconductor medium, for example, a solid-state drive.

In embodiments of this application, unless otherwise stated or there is a logic conflict, terms and/or descriptions in different embodiments are consistent and may be mutually referenced, and technical features in different embodiments may be combined based on an internal logical relationship thereof, to form a new embodiment.

It may be understood that various numbers in embodiments of this application are merely used for distinguishing for ease of description, and are not used to limit the scope of embodiments of this application. Sequence numbers of the foregoing processes do not mean an execution sequence, and the execution sequence of the processes should be determined based on functions and internal logic of the processes.

## Claims

1. A service processing method, applied to a terminal device, wherein the method comprises:
determining, at a first moment, whether a first service is in an enabled state, wherein the first service is a service for periodically enabling a first sensor to collect data, an execution periodicity of the first service is a first periodicity, and the first sensor comprises a target sensor;
if the first service is in the enabled state at the first moment, periodically executing the first service by using the first moment as a start moment;
determining, at the first moment, whether a second service is in an enabled state, wherein the second service is a service for periodically enabling a second sensor to collect data, an execution periodicity of the second service is a second periodicity, and the second sensor comprises the target sensor; and
if the second service is in the enabled state at the first moment, periodically executing the second service by using the first moment as a start moment.

2. The method according to claim 1, wherein the second periodicity is greater than the first periodicity, and the method further comprises:
if the first service is not in the enabled state at the first moment, determining, at a second moment, whether the first service is in the enabled state, wherein a duration between the second moment and the first moment is equal to a first duration, and the first duration is equal to a greatest common divisor of a duration of the first periodicity and a duration of the second periodicity.

3. The method according to claim 2, wherein the method further comprises:
if the second service is not in the enabled state at the first moment, determining, at a third moment, whether the second service is in the enabled state; and
if the second service is in the enabled state at the third moment, periodically executing the second service by using the third moment as a start moment.

4. The method according to claim 3, wherein the method further comprises:
if the second service is not in the enabled state at the third moment and it is determined that the second service is not in the enabled state at each of M moments, determining, at a fourth moment, whether the second service is in the enabled state, wherein an interval between the fourth moment and the third moment is a duration of one second periodicity, M is a positive integer, a duration of an interval between an m^{th} moment in the M moments and the first moment is equal to m first durations, m is a positive integer and ranges from 1 to M, an M^{th} moment in the M moments is before the fourth moment, an (M+1)^{th} moment is at the fourth moment, and a duration of an interval between the (M+1)^{th} moment and the first moment is equal to (M+1) first durations.

5. The method according to any one of claims 1 to 4, wherein the method further comprises:
if both the first service and the second service are in the enabled state at a target moment, determining a priority of the first service and a priority of the second service based on at least one of a first condition, a second condition, and a third condition, wherein the first condition is that a service that requires a larger quantity of sensors has a higher priority, the second condition is that a service with more preset data reporting frequencies of a sensor has a higher priority, the third condition is that a service with a shorter preset service running duration has a higher priority, a determining priority of the first condition is higher than a determining priority of the second condition, and the determining priority of the second condition is higher than a determining priority of the third condition; and
determining an execution rank of the first service and an execution rank of the second service in descending sequence of the priority of the first service and the priority of the second service.

6. The method according to claim 5, wherein determining the execution rank of the first service and the execution rank of the second service in descending sequence of the priority of the first service and the priority of the second service comprises:
if the priority of the first service is higher than the priority of the second service, determining to enable the first sensor at the target moment, and enable a sensor in the second sensor other than the target sensor after the target moment; or
if the priority of the first service is lower than the priority of the second service, determining to enable the second sensor at the target moment, and enable a sensor in the first sensor other than the target sensor after the target moment.

7. The method according to claim 5 or 6, wherein the method further comprises:
determining a target data reporting frequency based on a preset data reporting frequency of the first service and a preset data reporting frequency of the second service, wherein the target data reporting frequency is equal to a greatest common divisor of the preset data reporting frequency of the first service and the preset data reporting frequency of the second service; and
determining the target data reporting frequency as a data reporting frequency of the target sensor after the target moment.

8. The method according to claim 7, wherein the method further comprises:
determining a target service from the first service and the second service, wherein the target service is a service with a shorter preset service running duration in the first service and the second service;
if a preset service running duration of the target service is shorter than a preset service running duration of another service that is not the target service in the first service and the second service, determining an end moment of the target service based on the preset service running duration of the target service, and an interval between the end moment and the target moment is equal to a preset service duration of the target service; and
determining a preset data reporting frequency of the another service as a data reporting frequency of the target sensor after the end moment.

9. A service processing method, applied to a terminal device, wherein the method comprises:
determining, by a processor of the terminal device, that a to-be-processed service is in an enabled state at a target moment, wherein the to-be-processed service is a service for periodically enabling a third sensor to collect data, and the to-be-processed service comprises a first service and/or a second service; and
when determining to execute the to-be-processed service, sending, by the processor, indication information to the third sensor, wherein the indication information indicates the third sensor to collect and report the data.

10. The method according to claim 9, wherein when the to-be-processed service comprises the first service and the second service, the third sensor comprises a first sensor and a second sensor, the first service is a service for periodically enabling the first sensor to collect data, the second service is a service for periodically enabling the second sensor to collect data, and both the first sensor and the second sensor comprise a target sensor; and
sending, by the processor, the indication information to the third sensor comprises:
sending, by the processor, the indication information to the first sensor and the second sensor based on a priority of the first service and a priority of the second service.

11. The method according to claim 10, wherein sending, by the processor, the indication information to the first sensor and the second sensor based on the priority of the first service and the priority of the second service comprises:
if the priority of the first service is higher than the priority of the second service, sending, by the processor, first indication information to the first sensor, wherein the first indication information indicates the first sensor to collect first data and upload the first data at the target moment; and
sending, by the processor, second indication information to a sensor in the second sensor other than the target sensor, wherein the second indication information indicates the another sensor to collect second data and upload the second data after the target moment; or
if the priority of the first service is lower than the priority of the second service, sending, by the processor, third indication information to the second sensor, wherein the third indication information indicates the second sensor to collect second data and upload the second data at the target moment; and
sending, by the processor, fourth indication information to a sensor in the first sensor other than the target sensor, wherein the fourth indication information indicates the another sensor to collect first data and upload the first data after the target moment.

12. The method according to claim 10 or 11, wherein the method further comprises:
sending, by the processor, fifth indication information to the target sensor, wherein the fifth indication information indicates the target sensor to report data based on a target data reporting frequency after the target moment, and the target data reporting frequency is equal to a greatest common divisor of a preset data reporting frequency of the first service and a preset data reporting frequency of the second service.

13. The method according to claim 12, wherein the method further comprises:
sending, by the processor, sixth indication information to the target sensor, wherein the sixth indication information indicates the target sensor to report data based on a preset data reporting frequency of another service after an end moment of a target service, the target service is a service with a shorter preset service running duration in the first service and the second service, and the another service is a service in the first service and the second service other than the target service.

14. A service processing method, applied to a terminal device, wherein the method comprises:
receiving, by a first processor of the terminal device, service state information, wherein the service state information indicates that a to-be-processed service is in an enabled state at a target moment, the to-be-processed service is a service for periodically enabling a third sensor to collect data, and the to-be-processed service comprises a first service and/or a second service;
when determining to execute the to-be-processed service, sending, by the first processor, first information to a second processor, wherein the first information indicates to execute the to-be-processed service;
receiving, by the first processor, indication information from the second processor, wherein the indication information indicates the third sensor to collect and report the data; and
sending, by the first processor, the indication information to the third sensor.

15. The method according to claim 14, wherein when the to-be-processed service comprises the first service and the second service, the third sensor comprises a first sensor and a second sensor, the first service is a service for periodically enabling the first sensor to collect data, the second service is a service for periodically enabling the second sensor to collect data, and both the first sensor and the second sensor comprise a target sensor; and
sending, by the first processor, the indication information to the third sensor comprises:
sending, by the first processor, the indication information to the first sensor and the second sensor based on a priority of the first service and a priority of the second service.

16. The method according to claim 15, wherein sending, by the first processor, the indication information to the first sensor and the second sensor based on the priority of the first service and the priority of the second service comprises:
if the priority of the first service is higher than the priority of the second service, sending, by the first processor, first indication information to the first sensor, wherein the first indication information indicates the first sensor to collect first data and upload the first data at the target moment; and
sending, by the first processor, second indication information to a sensor in the second sensor other than the target sensor, wherein the second indication information indicates the another sensor to collect second data and upload the second data after the target moment; or
if the priority of the first service is lower than the priority of the second service, sending, by the first processor, third indication information to the second sensor, wherein the third indication information indicates the second sensor to collect second data and upload the second data at the target moment; and
sending, by the first processor, fourth indication information to a sensor in the first sensor other than the target sensor, wherein the fourth indication information indicates the another sensor to collect first data and upload the first data after the target moment.

17. The method according to claim 15 or 16, wherein the method further comprises:
sending, by the first processor, fifth indication information to the target sensor, wherein the fifth indication information indicates the target sensor to report data based on a target data reporting frequency after the target moment, and the target data reporting frequency is equal to a greatest common divisor of a preset data reporting frequency of the first service and a preset data reporting frequency of the second service.

18. The method according to claim 17, wherein the method further comprises:
sending, by the first processor, sixth indication information to the target sensor, wherein the sixth indication information indicates the target sensor to report data based on a preset data reporting frequency of another service after an end moment of a target service, the target service is a service with a shorter preset service running duration in the first service and the second service, and the another service is a service in the first service and the second service other than the target service.

19. An apparatus, comprising a functional module configured to implement the method according to any one of claims 1 to 8, or comprising a functional module configured to implement the method according to any one of claims 9 to 13, or comprising a functional module configured to implement the method according to any one of claims 14 to 18.

20. An apparatus, comprising a memory and a processor, wherein
the memory is configured to store program instructions; and
the processor is configured to execute the program instructions in the memory to implement the method according to any one of claims 1 to 8, or is configured to execute the program instructions in the memory to implement the method according to any one of claims 9 to 13, or is configured to execute the program instructions in the memory to implement the method according to any one of claims 14 to 18.

21. A computer-readable storage medium, wherein the computer-readable storage medium stores program code to be executed by a computer, and the program code comprises instructions used to implement the method according to any one of claims 1 to 8, any one of claims 9 to 13, or any one of claims 14 to 18.

22. A computer program product, wherein the computer program product comprises instructions used to implement the method according to any one of claims 1 to 8, any one of claims 9 to 13, or any one of claims 14 to 18.
